(19) Europäisches Patentamt
European Patent Office
Office européen des brevets

(11) EP 4 310 128 A1

(12) **EUROPEAN PATENT APPLICATION**
published in accordance with Art. 153(4) EPC

(43) Date of publication:
**24.01.2024 Bulletin 2024/04**

(21) Application number: **22771080.3**

(22) Date of filing: **28.02.2022**

(51) International Patent Classification (IPC):
***C08G 59/68*** *(2006.01)* ***C08G 65/10*** *(2006.01)*
***C08G 65/18*** *(2006.01)* ***A61K 6/60*** *(2020.01)*
***A61K 6/62*** *(2020.01)*

(52) Cooperative Patent Classification (CPC):
**A61K 6/60; A61K 6/62; C08G 59/68; C08G 65/10; C08G 65/18**

(86) International application number:
**PCT/JP2022/008296**

(87) International publication number:
**WO 2022/196326 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2021 JP 2021041639**
**16.09.2021 JP 2021150933**
**14.02.2022 JP 2022020842**
**14.02.2022 JP 2022020843**

(71) Applicant: **Tokuyama Dental Corporation**
**Tokyo 110-0016 (JP)**

(72) Inventors:
- **MIYAKE, Hideaki**
**Tokyo 110-0016 (JP)**
- **TAGO, Moeno**
**Tokyo 110-0016 (JP)**
- **AKIZUMI, Hironobu**
**Tokyo 110-0016 (JP)**
- **KIRA, Ryuta**
**Tokyo 110-0016 (JP)**
- **NOMURA, Naoto**
**Tokyo 110-0016 (JP)**

(74) Representative: **Hoffmann Eitle**
**Patent- und Rechtsanwälte PartmbB**
**Arabellastraße 30**
**81925 München (DE)**

# (54) PHOTOCATIONICALLY CURABLE COMPOSITION

(57) The photocationically curable composition of the present invention contains a cationically polymerizable monomer (a), a photo acid generator (b), a photosensitizer (c), and a thiophene compound (d). The present invention can provide a photocationically curable composition that can be rapidly cured at room temperature, can provide a cured material having high color tone stability, and is relatively resistant to gelation.



Processed by Luminess, 75001 PARIS (FR)

## Description

### Technical Field

The present invention relates to a photocationically curable composition. In more detail, the present invention relates to a composition that is rapidly photocured and also is excellent in color tone stability and storage stability.

### Background Art

In recent years, a photocurable type resin composition has been frequently used for adhesion of electric or electronic components, and also as a paint composition and the like. The photocurable type resin composition has many advantages, for example, it is more rapidly cured than a thermally curable type adhesive material, and does not deliver thermal damage to the adherend since it is not heated, the working time until curing can be arbitrarily set since it is not cured without irradiation with light, and no solvent or the like is evaporated in curing.

The photocurable type resin composition is roughly classified into a radical polymerization type and a cationic polymerization type, and a radical polymerization type using a (meth)acrylic-based polymerizable compound having good curability has been mainly developed and used.

The photocurable resin composition is also applied to the dental use. Examples thereof include a photocurable filling and restorative material referred to as a composite resin used for repairing damaged teeth due to caries, fracture, or the like, a lining material of a denture base, and hybrid ceramics for repairing a dental crown, and the composition has been used widely due to the convenience in handling. The photocurable dental material of this type is generally formed of a polymerizable monomer and a polymerization initiator, and for the composite resin and the hybrid ceramics, further contains a filler. As the polymerizable monomer, a (meth)acrylate-based radically polymerizable monomer has been used due to the good photopolymerizability thereof.

However, the (meth)acrylate-based radically polymerizable monomer has a problem in large polymerization shrinkage. Specifically, after filling a cavity of a tooth to be repaired with a filling and restorative material, such as a composite resin, the surface of the filling and restorative material is irradiated with light for polymerizing and curing, and the shrinkage due to polymerization causes a stress releasing from the interface to the tooth, resulting in a tendency that a gap tends to occur between the tooth and the filling and restorative material. Accordingly, there has been a demand of a curable composition that has polymerization shrinkage as small as possible, causing less gap in curing.

As a polymerizable monomer having small polymerization shrinkage, a cationically polymerizable monomer, such as an epoxide and a vinyl ether, has been known. However, the photoradical polymerization initiator that has been applied to the dental use, such as an $\alpha$-diketone compound and an acylphosphine oxide-based compound, cannot polymerize the cationically polymerizable monomer, and therefore a photocationic polymerization initiator is required.

The photocationic polymerization initiator is generally formed of a photo acid generator and a photosensitizer. The photo acid generator generally used includes an onium salt compound, such as an iodonium salt compound, a sulfonium salt compound, and a pyridinium salt compound. The photosensitizer used include a ketone compound (particularly an $\alpha$-diketone compound) and a coumarin-based colorant compound. For example, the application of a photocationic polymerization initiator formed of an iodonium salt and an $\alpha$-dicarbonyl compound to the dental use has been reported (see PTL 1).

In the case where the photocationically curable composition is applied to the dental use, it is necessary to proceed the polymerization rapidly under room temperature condition. Therefore, a photocationic polymerization initiator having higher activity is demanded. For enhancing the activity of the photocationic polymerization initiator, there has been a method of adding an electron donating compound as the third component. For example, the enhancement of the cationic polymerization activity by the addition of an aromatic amine, an alkyl aryl polyether, or an anthracene compound as the electron donating compound has been reported (see PTLs 2 to 4).

The estimated reaction mechanism of the enhancement of the polymerization activity through the addition of an aromatic amine or an anthracene compound has been described in known literatures. For example, for the aromatic amine, the mechanism in which the aromatic amine donates an electron to camphorquinone as the photosensitizer to form a radical cation releasing a hydrogen ion has been reported (see NPL 1).

For the anthracene compound, the mechanism in which the anthracene compound donates an electron to an iodonium cation as a photo acid generator to form a radical cation, which then becomes a cation through combination with another radical species, releasing a hydrogen ion has been reported (see NPL 2).

The aromatic amine and anthracene oxidants have structures constituting the skeletons of an aniline dye and an anthracene dye, respectively, and therefore the aromatic amine and the anthracene compound may exhibit strong coloration under oxidative condition. Therefore, the photocationic polymerization initiator system using the aromatic amine or the anthracene compound is considered to have high coloration risk, and the present inventors have actually confirmed the occurrence of coloration. On the other hand, the coloration is necessarily suppressed in the case where

the photocationically curable composition is applied to the dental use. In the repairing remedy with a dental material, in general, the dental material after curing is demanded to have a color close to the natural teeth and to retain the color since the remedied part is demanded to be indistinctive.

Citation List

Patent Literatures

PTL 1: JPH10-508067A
PTL 2: JPH11-130945A
PTL 3: JP2001-520758A
PTL 4: JP2005-523348A

Non-patent Literatures

NPL 1: "Evaluation of Initiator Systems for Controlled and Sequentially Curable Free-Radical/Cationic Hybrid Photopolymerizations", Joe D. Oxman, Dwight W. Jacobs, Matthew C. Trom, Vishal Sipani, Beth Ficek, Alec B. Scranton, Journal of Polymer Science: Part A: Polymer Chemistry, 2005, 43, 1747-1756
NPL 2: " Molecular Design and Function of Photo-acid Generators Utilized for Advanced Industries ", Tomotaka Tsuchimura, Journal of Synthetic Organic Chemistry, Japan, 2020, 78, 41-54

Summary of Invention

Technical Problem

As described above, the addition of an aromatic amine and an anthracene compound as an electron donating compound is effective from the standpoint of the enhancement of the curing rate of the photocationically curable composition. However, according to the investigations by the present inventors, it has been confirmed that the photocationically curable composition having an aromatic amine or an anthracene compound added thereto is colored yellow in curing. Furthermore, in the continuous observation of the cured materials, it has been confirmed that the coloration gradually fades with the lapse of time.
The temporal change in color tone of the cured material causes a problem of failing to achieve the long-term color tone compatibility.
According to the investigations by the present inventors, furthermore, the use of an anthracene compound may cause earlier gelation of the composition than the case where the compound is not added. While the mechanism therefor is not clarified, it is considered that the anthracene compound accelerates the decomposition of the acid generator, which accelerates the progress of the cationic polymerization in storing. There is a possibility that the anthracene compound capable of reacting with oxygen in the air promotes the formation of radical species, thereby the decomposition reaction of the acid generator is accelerated. The photocationically curable composition is demanded to retain the properties thereof until irradiation with light, and therefore it is necessary to avoid the acceleration of gelation.
Under the circumstances, an object of the present invention is to provide a photocationically curable composition that is rapidly cured under room temperature condition, provides a cured material having high color tone stability, and is difficult to gel in storing.

Solution to Problem

The present inventors have made earnest investigations for solving the problem. As a result, it has been found that the use of a thiophene compound as an electron donating compound can provide a photocationically curable composition that can be rapidly cured at room temperature, can provide a cured material having high color tone stability, and has a property relatively resistant to gelation, and thus the present invention has been completed.
Specifically, a first embodiment of the present invention is a photocationically curable composition containing a cationically polymerizable monomer (a), a photo acid generator (b), a photosensitizer (c), and a thiophene compound (d).
In the photocationically curable composition of the aforementioned embodiment (which may be hereinafter referred to as a "photocationically curable composition of the present invention"), a content of an electron donating compound containing an anthracene compound and/or an aromatic amine compound is preferably 0.2 part by mass or less per 1 part by mass of the thiophene compound (d).

The thiophene compound (d) is preferably a compound having one or more $\pi$-conjugated substituent on a thiophene skeleton, and in this case, the $\pi$-conjugated substituent is more preferably one or more benzene ring or thiophene ring. The thiophene compound (d) is particularly preferably a compound represented by the following formula (1) or (2).

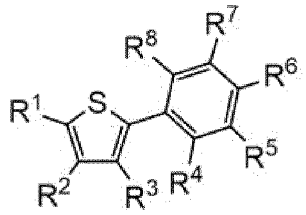

(1)

or

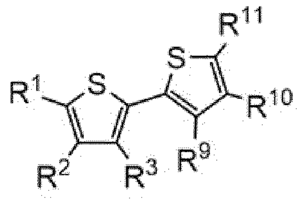

(2)

In the formulae (1) and (2), $R^1$ to $R^{11}$ each independently represent a hydrogen atom, a halogen atom, an alkyl group, an alkenyl group, an alkynyl group, an alkoxy group, an alkylthio group, an aryl group, or a thienyl group, in which $R^3$ and $R^4$, and $R^3$ and $R^9$ each may be bonded to each other to form a ring.

It is preferred that the cationically polymerizable monomer (a) contains at least one kind of a compound selected from an epoxy compound and an oxetane compound, the photo acid generator (b) is an iodonium salt, and the photosensitizer (c) is a camphorquinone, and the photocationically curable composition preferably further contains a phenol-based antioxidant, and also preferably further contains a filling material. The photocationically curable composition preferably contains a radically polymerizable monomer in an amount of 10 to 70 parts by mass per 100 parts by mass of the cationically polymerizable monomer.

It is preferred that the filling material contains an inorganic spherical filler ($f_1$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and the inorganic spherical filler ($f_1$) has a refractive index $nF_a$ at 25°C that is larger than a refractive index nP at 25°C of the polymer of the cationically polymerizable monomer (a).

The difference between the refractive index $nF_a$ at 25°C of the inorganic spherical filler ($f_1$) and the refractive index nP at 25°C of the polymer of the cationically polymerizable monomer (a) is preferably 0.001 or more.

It is preferred that the filling material contains an organic-inorganic composite filler (cf), the organic-inorganic composite filler (cf) contains an organic resin matrix (m) and an inorganic spherical filler ($f_2$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and the inorganic spherical filler ($f_2$) has a refractive index $nF_b$ at 25°C that is larger than a refractive index nM at 25°C of the organic resin matrix (m).

The difference between the refractive index $nF_b$ at 25°C of the inorganic spherical filler ($f_2$) and the refractive index nM at 25°C of the organic resin matrix (m) is preferably 0.001 or more.

The refractive index $nF_b$ at 25°C of the inorganic spherical filler ($f_2$) is preferably larger than the refractive index nP at 25°C of the polymer of the cationically polymerizable monomer (a).

The difference between the refractive index $nF_b$ at 25°C of the inorganic spherical filler ($f_2$) and the refractive index nP at 25°C of the polymer of the cationically polymerizable monomer (a) is preferably 0.001 or more.

A second embodiment of the present invention is a dental curable composition containing the photocationically curable composition of the present invention (which may be hereinafter also referred to as a "dental curable composition of the present invention").

Advantageous Effects of Invention

The photocationically curable composition of the present invention not only has a feature of small polymerization shrinkage, which is a feature of a photocationically curable composition containing a cationically polymerizable monomer and a photocationic polymerization initiator, but through the addition of a thiophene compound as an electron donating compound, also has the properties that the photocationically curable composition can be rapidly cured at room temperature, can provide a cured material having high color tone stability, and has a property relatively resistant to gelation. Furthermore, in the case where a radically polymerizable monomer is added to the photocationically curable composition of the present invention in a certain range, the adhesion strength in adhering by using a known bonding material can also be improved.

The dental curable composition of the present invention is largely reduced in the polymerization shrinkage, as compared to the composition of a radically polymerizable monomer used in the ordinary dental materials, and also has the properties including the rapid curability and the color tone stability. Accordingly, it can be said that the dental curable composition is particularly suitable for the application of a dental restorative material, such as a composite resin.

Description of Embodiments

The photocationically curable composition of the present invention contains a cationically polymerizable monomer (a) and a photocationic polymerization initiator containing a photo acid generator (b) and a photosensitizer (c), and exhibits an effect of small polymerization shrinkage, which are the same features as the ordinary photocationically curable composition. The photocationically curable composition of the present invention has the biggest feature in that a thiophene compound (d) is contained as an essential component, resulting in the properties that the photocationically curable composition can be rapidly cured at room temperature but is resistant to gelation, and can provide a cured material having high color tone stability.

Although the present invention is not restricted by the theories in anyway, the present inventors are estimating as follows about the functional mechanism of the aforementioned effects obtained by the thiophene compound mixed therein. Specifically, it is estimated that the thiophene compound functions as an electron donating compound as similar to the aromatic amine and the anthracene compound, thereby highly activating the photocationic polymerization initiator, and prevents substances causing coloration in the curing process and temporal change in color tone from remaining.

In more detail, it has been well known that thiophene has been frequently used as a skeleton of a p-type organic semiconductor material, and is high in $\pi$-conjugation effect and rich in electron donating capability. Therefore, in the case where the thiophene compound is added to the photocationic polymerization initiator, it is considered that electron donating reaction, which is similar to the aromatic amine and the anthracene compound, is caused thereby through irradiation of light, and a corresponding cation radical is generated. As for the general oxidative coupling reaction of a thiophene compound, the reaction mechanism in which the compound forms a cation radical through electron migration, and then forms a new bond while releasing an acid has been proposed, and therefore it is considered that the similar reaction proceeds to release an acid in the composition of the present invention.

It has been known that the cation radicals formed from the aromatic amine and the anthracene compound are relatively stable. The investigations made by the present inventors reveal such a phenomenon that the cured material of the composition using the aromatic amine or the anthracene compound exhibits yellow coloration immediately after light irradiation, and then gradually discolored, and it is considered that the phenomenon is caused by the active species derived from the cation radical partially remaining in a certain period of time. Specifically, it is considered that the remaining active species exhibits strong coloration immediately after light irradiation, and with the lapse of time, the colored active species is changed to the colorless or less colored stable compound to cause the decoloration. In general, an active species of a cation radical or the like frequently exhibits coloration through absorption of visible light.

On the other hand, the oxidative coupling reaction of a thiophene compound generally proceeds rapidly, and therefore it is considered that a cation radical derived from a thiophene compound has a relatively short lifetime. It is considered consequently that the amount of the active species remaining immediately after light irradiation is relatively small, and thereby the coloration and the temporal change in color tone are reduced.

As described above, the major characteristic feature of the photocationically curable composition of the present inevntion is the thiophene compound (d) contained as an essential component, and the other components do not particularly differ from the ordinary photocationically curable compositions. The components constituting the photocationically curable composition of the present invention including these components will be described below.

1. Cationically Polymerizable Monomer (a)

In the photocationically curable composition of the present invention, the cationically polymerizable monomer (a) as a polymerizable component is not particularly limited, as far as being a compound that is polymerized by an acid formed through decomposition of the photo acid generator, and the compounds that have been used as a cationically polymerizable monomer in the ordinaryphotocationically curable compositions, such as an epoxy compound, an oxetane compound, a cyclic ether compound a vinyl ether compound, a bicyclic ortho ester compound, a cyclic acetal compound, a bicyclic acetal compound, and a cyclic carbonate, may be used with no particular limitation. The use of an epoxy compound and/or an oxetane compound is preferred from the standpoint of the good availability, the small volume shrinkage, and the high polymerization rate.

Specific examples of the epoxy compound that can be preferably used include a compound having one epoxy functional group, such as 1,2-epoxypropane, methyl glycidyl ether, cyclohexene oxide, exo-2,3-epoxynorbornene, 4-vinyl-1-cyclohexene-1,2-epoxide, limonene oxide, $\alpha$-pinene oxide, styrene oxide, (2,3-epoxypropyl)benzene, and phenyl glycidyl ether; a compound having two epoxy functional groups, such as 1,3-butadiene oxide, ethylene glycol diglycidyl

ether, diethylene glycol diglycidyl ether, triethylene glycol diglycidyl ether, 1,6-hexanediol diglycidyl ether, diglycidyl glutarate, 4-vinyl-1-cyclohexene diepoxide, limonene diepoxide, and methylbis[2-(7-oxabicyclo[4.1.0]hept-3-yl)ethyl] phenylsilane; and a compound having three or more epoxy functional groups, such as glycerol triglycidyl ether, trimethylolpropane triglycidyl ether, pentaerythritol triglycidyl ether, pentaerythritol tetraglycidyl ether, and dipentaerythritol hexaglycidyl ether.

Examples of the epoxy compound also include a compound having a cyclic siloxane or silsesquioxane structure that has an epoxy functional group, such as the following compounds. In the following structural formulae (provided that the structural formulae defining R are excluded), the bond attached with *1 shows the bonding site to the O atom of the adjacent repeating unit, and the bond attached with *2 shows the bonding site to the Si atom of the adjacent repeating unit.

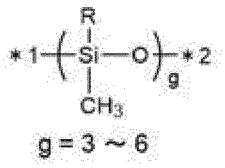

g = 3 ~ 6

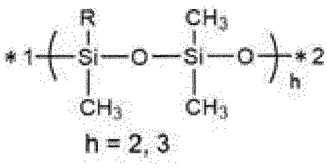

h = 2, 3

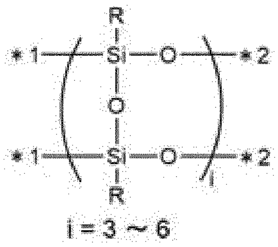

i = 3 ~ 6

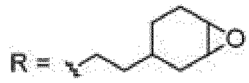

or

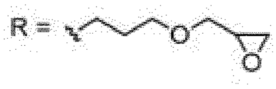

Among the epoxy compounds described above, a compound having two epoxy functional groups in one molecule is particularly preferably used from the standpoint of the properties of the resulting cured material.

Specific examples of the oxetane compound that can be preferably used include a compound having one oxetane ring, such as trimethylene oxide, 3-methyl-3-oxetanylmethanol, 3-ethyl-3-oxetanylmethanol, 3-ethyl-3-phenoxymethyloxetane, 3,3-diethyloxetane, and 3-ethyl-3-(2-ethylhexyloxy)oxetane, and a compound having two or more oxetane rings, such as 1,4-bis(3-ethyl-3-oxetanylmethyloxy)benzene, 4,4'-bis(3-ethyl-3-oxetanylmethyloxy)biphenyl, 4,4'-bis(3-ethyl-3-oxetanylmethyloxymethyl)biphenyl, ethylene glycol bis(3-ethyl-3-oxetanylmethyl) ether, diethylene glycol bis(3-ethyl-3-oxetanylmethyl) ether, and compounds represented by the following formulae.

In the bottom left structural formula in the structural formuale shown below, the bond attached with *1 shows the bonding site to the O atom of the adjacent repeating unit, and the bond attached with *2 shows the bonding site to the Si atom of the adjacent repeating unit.

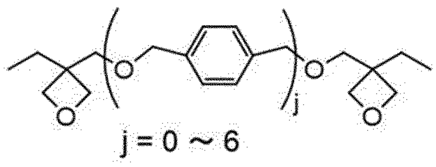

j = 0 ~ 6

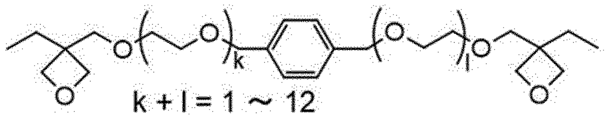

k + l = 1 ~ 12

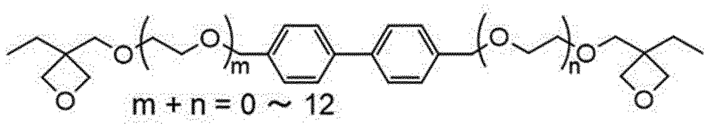

m + n = 0 ~ 12

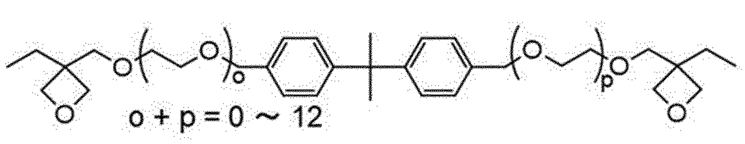

o + p = 0 ~ 12

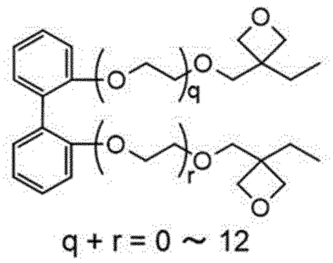

q + r = 0 ~ 12

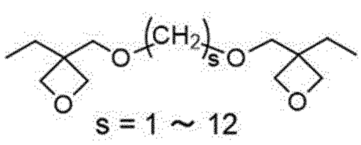

s = 1 ~ 12

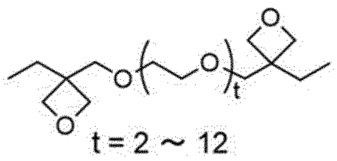

t = 2 ~ 12

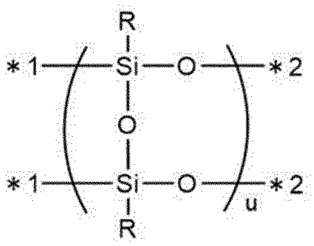

u = 3 ~ 6

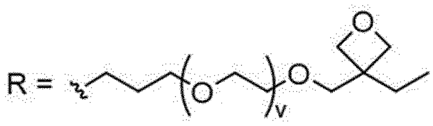

v = 0 ~ 6

Among the oxetane compounds described above, a compound having two or more oxetane rings in one molecule is particularly preferably used from the standpoint of the properties of the resulting cured material.

The cationically polymerizable monomer may be used alone, or two or more kinds thereof may be used in combination. In particular, a mixture prepared by mixing A mol of an oxetane compound having an oxetane functional group in a number of a in average per one molecule and B mol of an epoxy compound having an epoxy functional group in a number of b in average per one molecule to make $(a\times A)/(b\times B)$ within a range of 90/10 to 10 /90 is preferred since a high curing rate can be obtained, and the polymerization can be prevented from being hindered with water.

The cationically polymerizable monomer used may be a compound having a cationically polymerizable functional group and a radically polymerizable functional group in one molecule. Examples of the compound include glycidyl (meth)acrylate, 3,4-epoxycyclohexyl (meth)acrylate, 2-vinyloxyethyl (meth)acrylate, 6-vinyloxyhexyl (meth)acrylate, 3-ethyl-3-(meth)acryloxymethyloxetane, 3-ethyl-3-(2-(meth)acryloxyethyloxymethyl)oxetane, and p-(meth)acryloxymethylstyrene.

In the present invention, the cationically polymerizable monomer (a) used may be multiple kinds of polymerizable monomers for regulating the properties of the cured material (e.g., the mechanical characteristics and the adhesiveness to the tooth substance). In the case where the inorganic spherical filler ($f_1$) and/or the organic-inorganic composite filler (cf) described later are mixed therein, it is preferred to set the kinds and the mixing ratio of the polymerizable monomers to make a refractive index of the cationically polymerizable monomer (a) within a range of 1.38 to 1.55. In the case where the refractive index of the cationically polymerizable monomer (a) is set within a range of 1.38 to 1.55, the refractive index nP of the polymer obtained from the cationically polymerizable monomer (a) can be set within a range approximately of 1.40 to 1.57. There may be a case where multiple kinds of the cationically polymerizable monomers (a) are used, and as for the refractive index in this case, it suffices that the refractive index of the mixture is within the aforementioned range, and the refractive indices of the polymerizable monomers each may not necessarily be within the range. In the present invention, the refractive index is measured at 25°C with an Abbe refractometer.

2. Photo Acid Generator (b)

In the photocationically curable composition of the present invention, the photo acid generator (b) is not particularly limited, as far as being a compound generating a strong acid through reaction caused by light irradiation, and the compounds that have been used as a photo acid generator in the ordinary photocationically curable compositions, such as an iodonium salt compound, a sulfonium salt compound, a bismuthonium salt compound, and a pyridinium salt compound, may be used with no particular limitation. An iodonium salt compound is preferably used due to the good availability and the high polymerization activity thereof. The photo acid generator (b) may be used alone, or two or more kinds thereof may be used as a mixture.

Specific examples of the photo acid generator (b) that is preferably used include an onium salt compound having a cation or a cationic moiety and an anion or an anionic moiety respectively shown below.

[Cation or Cationic Moiety]

Examples thereof include diphenyl iodonium, bis(p-chlorophenyl) iodonium, ditolyl iodonium, bis(p-tert-butylphenyl) iodonium, p-isopropylphenyl-p-methylphenyl iodonium, bis(m-nitrophenyl) iodonium, p-tert-butylphenylphenyl iodonium, p-methoxyphenylphenyl iodonium, bis(p-methoxyphenyl) iodonium, p-octyloxyphenylphenyl iodonium, p-phenoxyphenylphenyl iodonium, bis(p-dodecylphenyl) iodonium, triphenyl sulfonium, tritolyl sulfonium, p-tert-butylphenyl diphenyl sulfonium, diphenyl 4-phenylthiophenyl sulfonium, diphenyl 2,4,6-trimethylphenyl sulfonium, tetraphenyl bismuthonium, triphenyl-2,4,6-trimethylphenyl bismuthonium, 1-methyl pyridinium, and 1-methyl 2-chloropyridinium.

[Anion or Anionic Moiety]

Examples thereof include tetrakispentafluorophenyl borate, tetra(nonafluoro-tert-butoxy) aluminate, hexafluoro phosphate, hexafluoro antimonate, hexafluoro arsenate, tetrafluoro borate, trifluoromethane sulfonate, and perchlorate.
The amount of the photo acid generator (b) used is not particularly limited, as far as being an amount capable of initiating the polymerization through light irradiation, and is generally 0.01 to 20 parts by mass, and preferably 0.05 to 10 parts by mass, per 100 parts by mass of the cationically polymerizable monomer (a), for the achievement of both the appropriate polymerization rate and the properties of the resulting cured material (such as the weather resistance and the hardness).

3. Photosensitizer (c)

In the photocationically curable composition of the present invention, the photosensitizer (c) is not particularly limited, as far as being a compound accelerating decomposition of the photo acid generator through absorption of light energy, and may be a ketone compound (particularly an $\alpha$-diketone compound), a coumarin-based colorant, a cyanine-based colorant, a merocyanine-based colorant, a thiazine-based colorant, an azine-based colorant, an acridine-based colorant, a xanthene-based colorant, a squalirium-based colorant, and a pyrylium salt-based colorant. Among these, an $\alpha$-diketone compound, such as camphorquinone, benzil, diacetyl, acetylbenzoyl, 2,3-pentadione, 2,3-octadione, 4,4'-dimethoxybenzil, 4,4'-oxybenzil, 9,10-phenanthrenequinone, and acenaphthenequinone, is preferably used, and among these, camphorquinone is particularly preferably used, from the standpoint of the suppression of coloration of the cured material.
The photosensitizer (c) may be used alone, or two or more kinds thereof may be used as a mixture, depending on necessity. The amount of the photosensitizer used may vary depending on the other components to be combined and the kind of the polymerizable monomer, and is generally 0.001 to 10 parts by mass, and preferably 0.01 to 5 parts by mass, per 100 parts by mass of the cationically polymerizable monomer (a).

4. Thiophene Compound (d)

In the photocationically curable composition of the present invention, the thiophene compound (d) used may be a known compound with no limitation, as far as being a compound having a thiophene ring in the molecule. In the present invention, the thiophene compound is used as an electron donating compound, and therefore a compound having a high electron donating capability is preferred. It has been known that the expansion of a $\pi$-conjugated system is effective for enhancing the electron donating capability. Therefore, a compound having a $\pi$-conjugated system substituent on the thiophene skeleton is preferably used. Examples of the partial structure constituting the $\pi$-conjugated system substituent include a benzene ring, and also include a heteroaryl ring, such as a thiophene ring, a furan ring, and a pyrrole ring. The thiophene compound in the present invention preferably has a $\pi$-conjugated system substituent, and it is particularly preferred that the $\pi$-conjugated system substituent has one or more of a benzene ring or a thiophene ring. The $\pi$-conjugated system constituted by thiophene and the partial structure may be substituted by a substituent, such as an alkyl group and an alkoxy group. Examples of the thiophene compound (d) of this type include a 2-phenylthiophene derivative, a 2,2'-bithiophene derivative, a 2-thienylfuran derivative, and a 2-thienylpyrrole derivative, and among these, a compound represented by the following general formula (1) or (2) is preferably used.

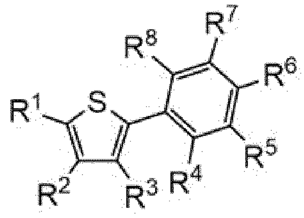

or

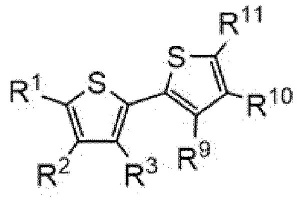

(1) (2)

In the formulae (1) and (2), $R^1$ to $R^{11}$ each independently represent a hydrogen atom, a halogen atom, an alkyl group, an alkenyl group, an alkynyl group, an alkoxy group, an alkylthio group, an aryl group, or a thienyl group, in which $R^3$ and $R^4$, and $R^3$ and $R^9$ each may be bonded to each other to form a ring. The alkyl group, the alkenyl group, the alkynyl group, the alkoxy group, and the alkylthio group are preferably an alkyl group, an alkenyl group, an alkynyl group, an alkoxy group, and an alkylthio group each having 1 to 12 carbon atoms, which may be linear or branched. The aryl group and the thienyl group each may have a substituent, such as an alkyl group, an alkenyl group, an alkynyl group, and an alkoxy group each having 1 to 12 carbon atoms, and a halogen atom, such as bromine. In the case where $R^3$ and $R^4$, and $R^3$ and $R^9$ each are bonded to each other to form a ring, the ring is preferably cyclopentadiene, thiophene, furan, cyclohexadiene, or benzene.

Specific examples of the compound represented by the general formula (1) or (2) include 2,5-diphenylthiophene, 2,3,4,5-tetraphenylthiophene, 2,2'-bithiophene, 5-hexyl-2,2'-bithiophene, 5-octyl-2,2'-bithiophene, 3,3'-dihexyl-2,2'-bithiophene, 4,4'-dihexyl-2,2'-bithiophene, 5,5'-dihexyl-2,2'-bithiophene, 2,2':5',2"-terthiophene, 5,5"-dibromo-2,2':5',2"-terthiophene, 4H-cyclopenta[2,1-b:3,4-b']dithiophene, and dithieno[3,2-b:2',3'-d]thiophene.

The thiophene compound (d) may be used alone, or two or more kinds thereof may be used as a mixture. The amount of the thiophene compound (d) used may vary depending on the other components to be combined and the kind of the polymerizable monomer, and may be 0.001 to 10 parts by mass, and preferably 0.01 to 5 parts by mass, per 100 parts by mass of the cationically polymerizable monomer (a). The photocationically curable composition of the present invention may contain an electron donating compound other than the thiophene compound (d) in such a range that does not adversely affect the effects. However, as for an electron donating compound consisting of an anthrathene compound and/or an aromatic amine compound, the content thereof is preferably 0.2 part by mass or less, and particularly preferably 0.1 part by mass or less, per 1 part by mass of the thiophene compound (d), and it is particularly preferred that the compound is not contained.

5. Other Components

The photocationically curable composition of the present invention may contain additional components depending on necessity in addition to the compoents described above in such kind and range of mixing amount that do not impair the effects of the present invention. The "additional components" will be described below.

5-1. Phenol-based Antioxidant

The photocationically curable composition of the present invention may contain a phenol-based antioxidant as an additive for suppressing decomposition of the photo acid generator. Conventionally known phenol-based antioxidants may be used with no limitation. Exmaples thereof include 4-methoxyphenol, hydroquinone, 2,6-di-t-butylphenol, dibutylhydroxytoluene, 2,4-di-t-butylphenol, and 2-t-butyl-4,6-dimethylphenol.

The phenol-based antioxidant described above may be used alone, or two or more kinds thereof may be used as a mixture, depending on necessity. The amount of the photosensitizer may vary depending on the other components to be combined and the kind of the polymerizable monomer, and may be 0.001 to 10 parts by mass, and preferably 0.01 to 5 parts by mass, per 100 parts by mass of the cationically polymerizable monomer (a).

5-2. Filling Material

In the case where the photocationically curable composition of the present invention is used as a dental filling and restorative material, a filling material (filler) is preferably mixed therein.

The filling material mixed therein may be any of an organic filler, an inorganic filler, and an organic-inorganic composite filler that have been mixed in dental filling and restorative materials, and examples of the organic filler include particles formed of an organic polymer, such as polymethyl methacrylate, polyethyl methacrylate, a methyl methacrylate-ethyl methacrylate copolymer, crosslinked polymethyl methacrylate, crosslinked polyethyl methacrylate, an ethylene-vinyl acetate copolymer, a styrene-butadiene copolymer, an acrylonitrile-styrene copolymer, and an acrylonitrile-butadiene-styrene copolymer.

Specific examples of the inorganic filler include inorganic particles of quartz, silica, alumina silica-titania, silica-zirconia, lanthanum glass, barium glass, and strontium glass. Examples of the organic-inorganic composite filler include an organic-inorganic composite filler obtained in such a manner that the inorganic particles are mixed with a polymerizable monomer in advance to form a paste, which is then polymerized and pulverized. The use of a filler containing a heavy metal, such as zirconia, as the inorganic filler, can also impart an X-ray contrast ability.

The particle diameter and the shape of the filling material are not particularly limited, and it suffices that spherical or irregular particles having an average particle diameter of 0.01 pm to 100 pm having been ordinarily used as a dental material are appropriately used depending on the target. The refractive index of the filling material is also not particularly limited, and a material having a refractive index of the ordinary filling material for a dental curable composition, which is in a range of 1.4 to 1.7, may be used with no limitation.

In the case where the filling material is mixed in the photocationically curable composition of the present invention, the mixing amount thereof is not particularly limited, as far as the composition becomes a paste, and in the case where the composition is used as a dental filling and restorative material, it is preferred that an organic filler and/or an organic-inorganic composite filler is used in an amount of 50 to 1,500 parts by mass, and preferably 70 to 1,000 parts by mass, per 100 parts by mass of the cationically polymerizable monomer (a).

The filling material, such as an organic filler and an organic-inorganic composite filler, may be used alone, or multiple kinds thereof that are different from each other in material, particle diameter, shape, and the like may be used in combination. In the case where the photocationically curable composition of the present invention is used as a dental curable composition, such as a dental filling and restorative material, an inorganic filler is particularly preferably used mainly from the standpoint of the excellent mechanical properties after curing.

An inorganic spherical filler ($f_1$) and/or an organic-inorganic composite filler (cf) shown below are preferably used from the standpoint of the achievement of restoration with high color tone compatibility with the natural teeth.

[Inorganic Spherical Filler (fi)]

The filling material may contain an inorganic spherical filler ($f_1$) having an average primary particle diameter in a range of 230 nm or more and 1,000 nm or less and a proportion of particles existing within a range above and below 5% of the average primary particle diameter in a number-based particle size distribution of 90% or more with respect to the total number of particles. In the case where the photocationically curable composition of the present invention is used as a dental curable composition, such as a dental filling and restorative material, the inorganic spherical filler ($f_1$) contained enables the restoration with high color tone compatibility with the natural teeth.

The inorganic spherical filler ($f_1$) is constituted by numerous primary particles, and 90% or more by number of the primary particles among the total number of the primary particles exist within a range above and below 5% of the average primary particle diameter of the primary particles (i.e., a range of ±5% of the value of the average primary particle diameter as 100%). The particles existing within a range above and below 5% of the average primary particle diameter are preferably 91% or more, and more preferably 93% or more, based on the total number of the particles.

The inorganic spherical filler ($f_1$) can create colored light by structural color based on interference, diffraction, refraction, scattering, etc. of light (which may be hereinafter referred simply to as "interference, scattering, etc.") due to the narrow particle size distribution thereof. The coloration by structural color utilizing interference, scattering, etc. is preferred since the decoloration and discoloration occurring in the use of a pigment substance, a dye substance, or the like can be avoided. Accordingly, the photocationically curable composition containing the inorganic spherical filler ($f_1$) has an advantage that a coloring substance, such as a pigment substance and a dye substance, having been used for regulating the color tone may not be used since the color tone compatibility with the natural teeth can be enhanced by utilizing colored light by structural color.

The colored light showing structural color exhibiting by interference, scattering, etc. is exhibited by strengthening light of the particular wavelength or scattering light other than the light of the particular wavelength, so as to reflect only the light of the particular wavelength, due to the occurrence of diffraction and interference according to the Bragg condition, and the inorganic spherical filler having the average primary particle diameter and the particle size distribution described above mixed therein allows the cured material of the photocationically curable composition to exhibit yellowish to reddish colored light corresponding to the average primary particle diameter. The average primary particle diameter of the inorganic spherical filler ($f_1$) is preferably 230 nm or more and 800 nm or less, more preferably 230 nm or more and 500 nm or less, and most preferably 260 nm or more and 350 nm or less, from the standpoint of the further enhancement of the exhibiting effect of the colored light by the interference, scattering, etc. In the case where an inorganic spherical filler having an average primary particle diameter of less than 230 nm is used, the coloration becomes bluish color, which does not match the color tone of the dentine. In the case where a spherical filler having an average primary

particle diameter of less than 100 nm is used, structural color is difficult to occur. In the case where an inorganic spherical filler having an average primary particle diameter exceeding 1,000 nm is used, on the other hand, the interference, scattering, etc. of light can be expected to occur, but the sedimentation and the abradability of the inorganic spherical filler are deteriorated, and therefore the filler is not preferred as the dental restorative material.

As described above, the average primary particle diameter of the inorganic spherical filler ($f_1$) is 230 nm to 1,000 nm, and the cured material of the photocationically curable composition exhibits yellowish to reddish colored light corresponding to the average primary particle diameter. In the case where the inorganic spherical filler having an average primary particle diameter in a range of 230 nm to 260 nm is used, the resulting colored light is yellowish color, which is useful for the restoration of a cavity of a tooth to be restored, the color tone of the tooth plane of the surrounding teeth of which is in the B series (reddish yellow) of the shade guide, "VITAPAN Classical (registered trademark)". In the case where the inorganic spherical filler having an average particle diameter in a range of 260 nm to 350 nm is used, the resulting colored light is reddish color, which is useful for the restoration of a cavity of a tooth to be restored, the color tone of the tooth plane of the surrounding teeth of which is in the A series (reddish brown) of the shade guide, "VITAPAN Classical (registered trademark)". The hue of the dentine is frequently reddish color of this type, and therefore an embodiment using the inorganic spherical filler having an average particle diameter of 260 nm to 350 nm is preferred since a wide range of compatibility can be obtained with teeth to be restored having various color tones.

It is important that the inorganic spherical filler ($f_1$) has an average primary particle diameter within the aforementioned range, and the primary particles may exist as aggregated particles in some extent, as far as the primary particles satisfy the requirement. However, the primary particles preferably exist as independent particles as much as possible, and specifically the amount of aggregated particles of 10 pm or more is preferably less than 10% by volume.

In the present invention, the average primary particle diameters of the inorganic spherical filler ($f_1$) and the inorganic spherical filler ($f_2$) described later are determined in such a manner that a photograph of the powder is taken with a scanning electron microscope, the number of all the particles (30 particles or more) observed within the unit view field of the photograph and the primary particle diameters (maximum diameters) of all the particles are measured, and the average value is calculated from the measured values according to the following expression.

$$\bar{X} = \frac{\sum_{i=1}^{n} X_i}{n} \text{ (number average)}$$

wherein n represents the number of the particles, and $X_i$ represents the primary particle diameter (maximum diameter) of the i-th particle.

In the present invention, the proportion (%) of particles existing within a range above and below 5% of the average primary particle diameter in the number-based particle size distribution of the inorganic spherical filler ($f_1$) is obtained in such a manner that the number of particles having a primary particle diameter (maximum diameter) outside a range of ±5% of the average primary particle diameter obtained above is measured in all the particles (30 particles or more) observed within the unit view field of the photograph, and the resulting value is subtracted from the number of all the particles to provide the number of particles within a range of ±5% of the average particle diameter within the unit view field of the photograph, from which the proportion is calculated according to the following expression.

Proportion (%) of particles existing within range above and below 5% of average primary particle diameter with respect to number of all particles = [(number of particles existing within range above and below 5% of average primary particle diameter in the unit view field of a photograph taken by a scanning electron microscope)/(number of all particles in the unit view field of a photograph taken by a scanning electron microscope)] × 100

The proportion (%) of particles existing within a range above and below 5% of the average primary particle diameter of the inorganic spherical filler ($f_2$) described later can also calculated in the same manner as in the inorganic spherical filler ($f_1$).

In the present invention, the spherical shape of the inorganic spherical filler may be a substantially spherical shape, and may not necessarily be a completely true sphere. In general, a photograph of the particles is taken with a scanning electron microscope, and for each of the particles (30 or more particles) existing in the unit view field, a value is obtained by dividing the particle diameter in the direction perpendicular to the maximum diameter by the maximum diameter, and the average of the values, which is the average evenness degree, may be 0.6 or more, and more preferably 0.8 or more. The upper limit value of the average evenness degree is 1 including the case where the shape is a completely true sphere. The average evenness degree can be obtained by the method described in Examples.

The inorganic spherical filler ($f_1$) used may be a material that has been used as a component of the ordinary curable composition in the dental field, as far as satisfying the aforementioned requiements of the average primary particle diameter and the particle diameter distribution, and specific examples thereof include inorganic powders of amorphous silica, silica-titanium group oxide-based composite oxide particles (such as silica-zirconia and silica-titania),

quartz, alumina, barium glass, zirconia, titania, lanthanoid, and colloidal silica. Among these, silica-titanium group oxide-based composite oxide particles are preferred since the refractive index can be easily regulated.

The silica-titanium group oxide-based composite oxide particles are a composite oxide of silica and a titanium group (i.e., a group IV element in the periodic table) oxide, and examples thereof include silica-titania, silica-zirconia, and silica-titania-zirconia. Among these, silica-zirconia is preferred since the refractive index of the filler can be regulated, and in addition, high X-ray impermeability can be imparted. While the composite ratio thereof is not particularly limited, a material having a content of silica of 70 to 95% by mol and a content of the titanium group oxide of 5 to 30% by mol is preferred from the standpoint that the sufficient X-ray impermeability is imparted, and the refractive index is allowed to be within the preferred range described later. Silica-zirconia can be freely changed in refractive index thereof by changing the composite ratio.

The silica-titanium group oxide-based composite oxide particles are permitted to contain a metal oxide other than silica and the titanium group oxide as a composite in a small amount. Specifically, an alkali metal oxide, such as sodium oxide and lithium oxide, may be contained within 10% by mol or less.

The production method of the silica-titanium group oxide-based composite oxide particles is not particularly limited, and for providing the particular inorganic spherical filler of the present invention, for exmaple, the so-called sol-gel method is preferably used, in which a mixed solution containing a hydrolyzable organic silicon compound and a hydrolyzable organic titanium compound is added to an alkaline solvent, so that hydrolysis is performed to deposit the reaction product.

The silica-titanium group oxide-based composite oxide particles may be surface-treated with a silane coupling agent. With the surface treatment with a silane coupling agent, an excellent interface strength to the polymer part of the polymerizable monomer component can be obtained. Representative examples of the silane coupling agent include an organic silicon compound, such as $\gamma$-methacryloyloxyalkyltrimethoxysilane and hexamethyldisilazane. The surface-treating amount of the silane coupling agent is not particularly limited, and may be determined to make an optimum value after confirming the mechanical properties and the like of the resulting photocationically curable composition by experiments in advance, and examples of the preferred range thereof include a range of 0.1 to 15 parts by mass per 100 parts by mass of the particles.

In the present invention, the refractive index $nF_a$ at 25°C of the inorganic spherical filler ($f_1$) is preferably larger than the refractive index nP at 25°C of the polymer of the cationically polymerizable monomer (a). According to the configuration, the exhibition of the good color tone compatibility with the natural teeth can be facilitated.

The difference between the refractive index $nF_a$ (25°C) of the inorganic spherical filler ($f_1$) and the refractive index nP (25°C) of the polymer of the cationically polymerizable monomer (a) is preferably 0.001 or more, more preferably 0.002 or more, and most preferably 0.005 or more. The colored light can be exhibited more brilliantly with higher transparency of the cured material, and therefore, the difference between the refractive index $nF_a$ (25°C) of the inorganic spherical filler ($f_1$) and the refractive index nP (25°C) of the polymer of the cationically polymerizable monomer (a) is preferably 0.1 or less, and more preferably 0.05 or less.

In the case where the inorganic spherical filler ($f_1$) and the organic-inorganic composite filler (cf) described later are used in combination, the refractive index $nF_a$ at 25°C of the inorganic spherical filler ($f_1$) is preferably larger than the refractive index nM at 25°C of the organic resin matrix (m) from the standpoint of the enhancement of the color tone compatibility. The difference between the refractive index $nF_a$ (25°C) at 25°C of the inorganic spherical filler ($f_1$) and the refractive index nM (25°C) at 25°C of the organic resin matrix (m) is preferably 0.001 or more, more preferably 0.002 or more, and most preferably 0.005 or more. The colored light can be exhibited more brilliantly with higher transparency of the cured material, and therefore, the difference between the refractive index $nF_a$ (25°C) of the inorganic spherical filler ($f_1$) and the refractive index nM (25°C) of the organic resin matrix (m) is preferably 0.1 or less, and more preferably 0.05 or less.

In the present invention, the content of the inorganic spherical filler ($f_1$) is preferably 50 to 1,500 parts by mass per 100 parts by mass of the cationically polymerizable monomer (a). In the case where 50 parts by mass or more of the inorganic spherical filler ($f_1$) is mixed therein, the favorable exhibition of the colored light by interference, scattering, etc. can be facilitated. The amount of the inorganic spherical filler ($f_1$) mixed therein is preferably 70 to 1,000 parts by mass, more preferably 100 to 1,300 parts by mass, and further preferably 150 to 1,000 parts by mass, per 100 parts by mass of the cationically polymerizable monomer (a).

In the inorganic spherical filler ($f_1$), the silica-based filler, particularly the silica-titanium group oxide-based composite oxide, has a refractive index in a range approximately of 1.45 to 1.58 corresponding to the content of the silica component. Accordingly, by setting the refractive index of the cationically polymerizable monomer (a) to the range described above (1.38 to 1.55), the inorganic spherical filler ($f_1$) having the desired refractive index can be easily selected. Consequently, the silica-titanium group oxide-based composite oxide having a suitable amount of the silica component (for example, silica-titania or silica-zirconia) is preferably used.

<Organic-Inorganic Composite Filler (cf)>

The filling material preferably contains an organic-inorganic composite filler (cf). The organic-inorganic composite filler (cf) contains an organic resin matrix (m) and an inorganic spherical filler ($f_2$), and in more detail, the inorganic spherical filler ($f_2$) formed of multiple primary particles is included in the organic resin matrix (m), and the organic resin matrix (m) covers the surface of the primary particles constituting the inorganic spherical filler ($f_2$).

The use of the organic-inorganic composite filler suppresses the thickening of the photocationically curable composition in the form of a paste, as compared, for example, to the case using a fine inorganic spherical filler alone, and also provide an advantage that the stickiness and the polymerization shrinkage of the cured material of the photocationically curable composition can be reduced.

The organic-inorganic composite filler (cf) contained in the photocationically curable composition of the present invention contains the inorganic spherical filler ($f_2$) described later, and thereby the restoration with high color tone compatibility with the natural teeth can be performed.

<Inorganic Spherical Filler ($f_2$)>

The organic-inorganic composite filler (cf) in the present invention contains the inorganic spherical filler ($f_2$), and the inorganic spherical filler ($f_2$) is an inorganic spherical filler having an average primary particle diameter in a range of 230 nm or more and 1,000 nm or less and a proportion of particles existing within a range above and below 5% of the average primary particle diameter in a number-based particle size distribution of 90% or more with respect to the total number of particles.

The inorganic spherical filler ($f_2$) is constituted by numerous primary particles, and 90% or more by number of the primary particles among the total number of the primary particles exist within a range above and below 5% of the average primary particle diameter of the primary particles (i.e., a range of ±5% of the value of the average primary particle diameter as 100%). The particles existing within a range above and below 5% of the average primary particle diameter are preferably 91% or more, and more preferably 93% or more, based on the total number of the particles.

The inorganic spherical filler ($f_2$) can create colored light by structural color based on interference, scattering, etc. of light due to the narrow particle size distribution thereof The coloration by structural color utilizing interference, scattering, etc. is preferred since the decoloration and decoloration occurring in the use of a pigment substance, a dye substance, or the like can be avoided. Accordingly, the photocationically curable composition has an advantage that a coloring substance, such as a pigment substance and a dye substance, having conventionally been used for regulating the color tone may not be used since the color tone compatibility with the natural teeth can be enhanced by utilizing colored light by structural color.

In the case where the inorganic spherical filler ($f_2$) is used, as similar to the inorganic spherical filler ($f_1$) described above, the cured material of the photocationically curable composition exhibits yellowish to reddish colored light corresponding to the average primary particle diameter thereof. The average primary particle diameter of the inorganic spherical filler ($f_2$) is preferably 230 nm or more and 800 nm or less, more preferably 230 nm or more and 500 nm or less, and most preferably 260 nm or more and 350 nm or less, from the standpoint of the further enhancement of the exhibiting effect of the colored light by the interference, scattering, etc. In the case where an inorganic spherical filler having an average primary particle diameter of less than 230 nm is used, the coloration becomes bluish color, which does not match the color tone of the dentine. In the case where an inorganic spherical filler having an average primary particle diameter of less than 100 nm is used, structural color is difficult to occur. In the case where a spherical filler having an average primary particle diameter exceeding 1,000 nm is used, on the other hand, the interference, scattering, etc. of light can be expected to occur, but the sedimentation and the abradability of the inorganic spherical filler are deteriorated, and therefore the filler is not preferred as the dental restorative material.

As described above, the average primary particle diameter of the inorganic spherical filler ($f_2$) is 230 nm to 1,000 nm, and the cured material of the photocationically curable composition exhibits yellowish to reddish colored light corresponding to the average primary particle diameter. In the case where the inorganic spherical filler having an average primary particle diameter in a range of 230 nm to 260 nm is used, the resulting colored light is yellowish, which is useful for the restoration of a cavity of a tooth to be restored, the color tone of the tooth plane of the surrounding teeth of which is in the B series (reddish yellow) of the shade guide, "VITAPAN Classical (registered trademark)". In the case where the inorganic spherical filler ($f_2$) having an average particle diameter in a range of 260 nm to 350 nm is used, the resulting colored light is reddish color, which is useful for the restoration of a cavity of a tooth to be restored, the color tone of the tooth plane of the surrounding teeth of which is in the A series (reddish brown) of the shade guide, "VITAPAN Classical (registered trademark)". The hue of the dentine is frequently reddish color of this type, and therefore an embodiment using the inorganic spherical filler having an average particle diameter of 260 nm to 350 nm is preferred since a wide range of compatibility can be obtained with teeth to be restored having various color tones.

In the case where the inorganic spherical filler ($f_1$) described above and the organic-inorganic composite filler

(cf) are used in combination, the colored light generated by the inorganic spherical filler ($f_1$) and the colored light generated by the inorganic spherical filler ($f_2$) contained in the organic-inorganic composite filler (cf) are preferably colored light with the same color. Accordingly, the average particle diameter of the inorganic spherical filler ($f_1$) and the average particle diameter of the inorganic spherical filler ($f_2$) contained in the organic-inorganic composite filler are preferably relatively close to each other, and the difference between the average particle diameters thereof is preferably 0 to 100 nm, more preferably 0 to 50 nm, and further preferably 0 to 30 nm.

The inorganic spherical filler ($f_2$) used may be a material that have been used as a component of the ordinary curable composition in the dental field, as far as satisfying the aforementioned requiements of the average primary particle diameter and the particle diameter distribution, and specific examples thereof include inorganic powders of amorphous silica, silica-titanium group oxide-based composite oxide particles (such as silica-zirconia and silica-titania), quartz, alumina, barium glass, zirconia, titania, lanthanoid, and colloidal silica. Among these, silica-titanium group oxide-based composite oxide particles are preferred since the refractive index can be easily regulated.

The silica-titanium group oxide-based composite oxide particles are a composite oxide of silica and a titanium group (i.e., a group IV element in the periodic table) oxide, and examples thereof include silica-titania, silica-zirconia, and silica-titania-zirconia. Among these, silica-zirconia is preferred since the refractive index of the filler can be regulated, and in addition, high X-ray impermeability can be imparted. While the composite ratio thereof is not particularly limited, a material having a content of silica of 70 to 95% by mol and a content of the titanium group oxide of 5 to 30% by mol is preferred from the standpoint that the sufficient X-ray impermeability is imparted, and the refractive index is allowed to be within the preferred range described later. Silica-zirconia can be freely changed in refractive index thereof by changing each of the composite ratios.

The silica-titanium group oxide-based composite oxide particles are permitted to contain a metal oxide other than silica and the titanium group oxide as a composite in a small amount. Specifically, an alkali metal oxide, such as sodium oxide and lithium oxide, may be contained within 10% by mol or less.

The production method of the silica-titanium group oxide-based composite oxide particles is not particularly limited, and for providing the particular inorganic spherical filler of the present invention, for exmaple, the so-called sol-gel method is preferably used, in which a mixed solution containing a hydrolyzable organic silicon compound and a hydrolyzable organic titanium compound is added to an alkaline solvent, so that hydrolysis is performed to deposit the reaction product.

The silica-titanium group oxide-based composite oxide particles may be surface-treated with a silane coupling agent. With the surface treatment with a silane coupling agent, an excellent interface strength to the polymer part of the polymerizable monomer component can be obtained. Representative examples of the silane coupling agent include an organic silicon compound, such as γ-methacryloyloxyalkyltrimethoxysilane and hexamethyldisilazane. The surface-treating amount of the silane coupling agent is not particularly limited, and may be determined to make an optimum value after confirming the mechanical properties and the like of the resulting photocationically curable composition by experiments in advance, and examples of the preferred range thereof include a range of 0.1 to 15 parts by mass per 100 parts by mass of the particles.

In the present invention, the refractive index $nF_b$ at 25°C of the inorganic spherical filler ($f_2$) is preferably larger than the refractive index nM at 25°C of the organic resin matrix (m). According to the configuration, the exhibition of the good color tone compatibility with the natural teeth can be facilitated.

The difference between the refractive index $nF_b$ (25°C) of the inorganic spherical filler ($f_2$) and the refractive index nM (25°C) of the organic resin matrix (m) is preferably 0.001 or more, more preferably 0.002 or more, and most preferably 0.005 or more. The colored light can be exhibited more brilliantly with higher transparency of the cured material, and therefore, the difference between the refractive index $nF_b$ (25°C) of the inorganic spherical filler ($f_2$) and the refractive index nM (25°C) of the organic resin matrix (m) is preferably 0.1 or less, and more preferably 0.05 or less.

The refractive index $nF_b$ at 25°C of the inorganic spherical filler ($f_2$) is preferably larger than the refractive index nP at 25°C of the polymer of the cationically polymerizable monomer (a). According to the configuration, the exhibition of the good color tone compatibility with the natural teeth can be facilitated.

The difference between the refractive index $nF_b$ (25°C) of the inorganic spherical filler ($f_2$) and the refractive index nP (25°C) of the polymer of the cationically polymerizable monomer (a) is preferably 0.001 or more, more preferably 0.002 or more, and most preferably 0.005 or more. The difference between the refractive index $nF_b$ (25°C) of the inorganic spherical filler ($f_2$) and the refractive index nP (25°C) of the polymer of the cationically polymerizable monomer (a) is preferably 0.1 or less, and more preferably 0.05 or less.

The content of the inorganic spherical filler ($f_2$) in the organic-inorganic composite filler (cf) is preferably 30 to 95% by mass. In the case where the content of the inorganic spherical filler ($f_2$) is 30% by mass or more, the colored light of the cured material of the photocationically curable composition can be favorably exhibited, and the mechanical strength thereof can also be sufficiently enhanced. A content of the inorganic spherical filler ($f_2$) exceeding 95% by mass is difficult to achieve operationally. The content of the inorganic spherical filler ($f_2$) in the organic-inorganic composite filler (cf) is more preferably 40 to 90% by mass.

In the inorganic spherical filler ($f_2$), the silica-based filler, particularly the silica-titanium group oxide-based composite oxide, has a refractive index in a range approximately of 1.45 to 1.58 corresponding to the content of the silica component. Accordingly, by setting the refractive index of the inorganic spherical filler ($f_2$) approximately to 1.45 to 1.58, the differences thereof from the refractive index of the organic resin matrix (m) and the refractive index of the cationically polymerizable monomer (a) can be easily regulated to the desired ranges of the present invention. Consequently, the silica-titanium group oxide-based composite oxide having a suitable amount of the silica component (for example, silica-titania or silica-zirconia) is preferably used.

<Organic Resin Matrix (m)>

The organic resin matrix (m) is not particularly limited, and is preferably a polymer of a polymerizable monomer ($\alpha$) containing at least any of a radically polymerizable monomer and a cationically polymerizable monomer. Accordingly, the organic resin matrix (m) may be formed of a polymer of a radically polymerizable monomer, may be formed of a polymer of a cationically polymerizable monomer, and may be formed of a polymer of both a radically polymerizable monomer and a cationically polymerizable monomer.

The polymerizable monomer ($\alpha$) preferably contains a polyfunctional polymerizable monomer having two or more polymerizable functional group in the molecule. The use of a polyfunctional polymerizable monomer can increase the polyerizability and can enhance the mechanical characteristics of the resulting organic-inorganic composite filler. Examples of the polyfunctional polymerizable monomer include a radically polymerizable monomer having two or more radically polymerizable functional groups and a cationically polymerizable monomer having two or more cationically polymerizable functional groups.

(Radically Polymerizable Monomer)

The radically polymerizable monomer contained in the polymerizable monomer ($\alpha$) constituting the organic resin matrix is not particularly limited, as far as being a monomer having a radically polymerizable functional group, such as a vinyl group, an allyl group, and a (meth)acrylate group, and is preferably a radically polymerizable monomer having a (meth)acrylate group. The (meth)acrylate group herein means an acrylate group or a methacrylate group.

Exmaples of the polymerizable monomer having a (meth)acrylate group include a monofunctional polymerizable monomer (I) having one radically polymerizable group and a polyfucntional polymerizable monomer (II) having two or more radically polymerizable functional groups.

Examples of the polyfunctional polymerizable monomer (II) include a bifunctioanl polymerizable monomer (II-a), a trifunctional polymerizable monomer (II-b), and a tetrafunctional polymerizable monomer (II-c).

Specific exmaples thereof are shown below.

<Monofunctional Polymerizable Monomer (I)>

Examples thereof include methyl (meth)acrylate, ethyl (meth)acrylate, n-butyl (meth)acrylate, 2-ethylhexyl (meth)acrylate, n-lauryl (meth)acrylate, n-stearyl (meth)acrylate, tetrahydrofurfuryl (meth)acrylate, glycidyl (meth)acrylate, methoxy ethylene glycol (meth)acrylate, methoxy diethylene glycol (meth)acrylate, methoxy triethylene glycol (meth)acrylate, methoxy polyethylene glycol (meth)acrylate, ethoxy ethylene glycol (meth)acrylate, ethoxy diethylene glycol (meth)acrylate, ethoxy triethylene glycol (meth)acrylate, ethoxy polyethylene glycol (meth)acrylate, phenoxy ethylene glycol (meth)acrylate, phenoxy diethylene glycol (meth)acrylate, phenoxy triethylene glycol (meth)acrylate, phenoxy polyethylene glycol (meth)acrylate, cyclohexyl (meth)acrylate, benzyl (meth)acrylate, isobornyl (meth)acrylate, and trifluoroethyl (meth)acrylate.

Examples thereof also include 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol(meth)acrylamide, N-hydroxyethyl(meth)acrylamide, and N,N-(dihydroxyethyl)(meth)acrylamide.

<Polyfunctional Polymerizable Monomer (II)>

Bifunctional Polymerizable Monomer (II-a)

Examples of the bifunctional polymerizable monomer (II-a) include an aromatic bifunctional polymerizable monomer (II-a-1) and an aliphatic bifunctional polymerizable monomer (II-a-2) shown below.

Examples of the aromatic bifunctional polymerizable monomer (II-a-1) include the following compounds:

2,2-bis(methacryloyloxyphenyl)propane, 2,2-bis[(3-methacryloyloxy-2-hydroxypropyloxy)phenyl]propane, 2,2-bis(4-methacryloyloxyphenyl)propane, 2,2-bis(4-methacryloyloxypolyethoxyphenyl)propane, 2,2-bis(4-methacryloyloxydiethoxyphenyl)propane, 2,2-bis(4-methacryloyloxytetraethoxyphenyl)propane, 2,2-bis(4-methacryloyloxypentaethoxyphenyl)propane, 2,2-bis(4-methacryloyloxydipropoxyphenyl)propane, 2-(4-methacryloyloxydiethoxyphenyl)-2-(4-methacryloyloxytriethoxyphenyl)propane, 2-(4-methacryloyloxydipropoxyphenyl)-2-(4-methacryloyloxyethoxyphenyl)propane, 2,2-bis(4-methacryloyloxypropoxyphenyl)propane, and 2,2-bis(4-methacryloyloxyisopropoxyphenyl)propane, and acrylates corresponding to these methacrylates;

a diadduct obtained through addition of a vinyl monomer having a hydroxy group, such as a methacrylate, e.g., 2-hydroxyethyl methacrylate, 2-hydroxypropyl methacrylate, and 3-chloro-2-hydroxypropyl methacrylate, or an acrylte corresponding to the methacrylate, and a diisocyanate compound having aromatic group, such as diisocyanatomethylbenzene and 4,4'-diphenylmethane diisocyanate; and

di(methacryloxyethyl)diphenylmethanediurethane.

Examples of the aliphatic bifunctional polymerizable monomer (II-a-2) include the following compounds:

ethylene glycol dimethacrylate, diethylene glycol dimethacrylate, triethylene glycol dimethacrylate, tetraethylene glycol dimethacrylate, neopentylglycol dimethacrylate, 1,3-butanediol dimethacrylate, 1,4-butanediol dimethacrylate, and 1,6-hexanediol dimethacrylate, and acrylates corresponding to the methacrylates;

1,6-bis(methacrylethyloxycarbonylamino)trimethylhexane, and the like, and a diadduct obtained through addition of a vinyl monomer having a hydroxy group, such as a methacrylate, e.g., 2-hydroxyethyl methacrylate, 2-hydroxypropyl methacrylate, and 3-chloro-2-hydroxypropyl methacrylate, or an acrylte corresponding to the methacrylate, and a diisocyanate compound, such as hexamethylene diisocyanate, trimethylhexamethylene diisocyanate, diisocyanatomethylcyclohexane, isophorone diisocyanate, and methylenebis(4-cyclohexyl isocyanate); and

1,2-bis(3-methacryloyloxy-2-hydroxypropoxy)ethyl.

Trifunctional Polymerizable Monomer (II-b)

Examples of the trifunctional polymerizable monomer (II-b) include the following compounds.

Examples thereof include trimethylolpropane trimethacrylate, trimethyloethane trimethacrylate, pentaerythritol trimethacrylate, and trimethylolmethane trimethacrylate, and acrylates corresponding to these methacrylates.

Tetrafunctional Polymerizable Monomer (II-c)

Examples of the tetrafunctional polymerizable monomer (II-c) include the following compounds:

pentaerythritol tetramethacrylate and pentaerythritol tetraacrylate; and

a diadduct obtained from an adduct of a diisocyanate compound, such as diisocyanatomethylbenzene, diisocyanatomethylcyclohexane, isophorone diisocyanate, hexamethylene diisocyanate, trimethylhexamethylene diisocyanate, methylenebis(4-cyclohexyl isocyanate), 4,4-diphenylmethane diisocyanate, and tolylene 2,4-diisocyanate, and glycidol dimethacrylate.

In the case where the radically polymerizable monomer is contained in the polymerizable monomer ($\alpha$) constituting the organic resin matrix, the polyfunctional polymerizable monomer having two or more radically polymerizable functional groups is preferably contained therein, among the radically polymerizable monomers above.

One kind of the radically polymerizable monomer may be used alone, or two or more kinds thereof may be used in combination.

(Cationically Polymerizable Monomer)

The cationically polymerizable monomer contained in the polymerizable monomer ($\alpha$) constituting the organic resin matrix may be the compounds described for the cationically polymerizable monomer (a) described above contained in the photocationically curable composition of the present invention with no particular limitation.

In the case where the cationically polymerizable monomer is contained in the polymerizable monomer ($\alpha$), at least the polyfunctional polymerizable monomer having two or more cationically polymerizable functional groups or the compound having a cationically polymerizable functional group and a radically polymerizable functional group in the same molecule is preferably contained.

In the case where the polymerizable monomer ($\alpha$) contains both the radically polymerizable monomer and the cationically polymerizable monomer, the cationically polymerizable monomer used is preferably the compound having

a cationically polymerizable functional group and a radically polymerizable functional group in the same molecule.

Examples of the polyfunctional polymerizable monomer having two or more cationically polymerizable functional groups include the epoxy compound having two or more epoxy functional groups and the oxetane compound having two or more oxetane rings described above.

One kind of the cationically polymerizable monomer may be used alone, or two or more kinds thereof may be used in combination.

The difference between the refractive index nM at 25°C of the organic resin matrix (m) and the refractive index nP at 25°C of the polymer of the cationically polymerizable monomer (a) is preferably 0.007 or less, and more preferably 0.005 or less, from the standpoint of the transparency of the resulting cured material of the photocationically curable composition. In the case where the difference between the refractive index nM and the refractive index nP is regulated in this manner, there is a tendency that the transparency is enhanced, and the attenuation of the colored light by the interference, scattering, etc. is suppressed. The difference between the refractive index nM and the refractive index nP is preferably 0.001 or more from the standpoint that the light diffuseness can be imparted by the difference in refractive index, and the color tone compatibility of the cured material of the photocationically curable composition with the teeth can be enhanced.

The organic-inorganic composite filler (cf) may be a porous organic-inorganic composite filler. The porous organic-inorganic composite filler preferably has a cumulative pore volume of pores of 1 to 500 nm measured by the nitrogen adsorption method of 0.01 to 0.30 $cm^3/g$, more preferably 0.01 to 0.20 $cm^3/g$, and further preferably 0.03 to 0.15 $cm^3/g$. The average pore diameter of the porous organic-inorganic composite filler is preferably 3 to 300 nm, and more preferably 10 to 200 nm.

The production method of the organic-inorganic composite filler (cf) is not particularly limited, and an ordinary production method may be used, for exmaple, the components including the prescribed amounts of the inorganic spherical filler ($f_2$), the polymerizable monomer ($\alpha$) containing at least any of the radically polymerizable monomer and the cationically polymerizable monomer, and a polymerization initiator are mixed, polymerized by such a method as heating or light irradiation, and then pulverized. In alternative, the production method described in WO2011/115007 or WO2013/039169 may be used. In this production method, inorganic aggregated particles formed through aggregation of the inorganic spherical filler ($f_2$) are immersed in a polymerizable monomer solvent containing the polymerizable monomer, a polymerization initiator, and an organic solvent, and then the organic solvent is removed, and the polymerizable monomer is polymerized and cured by such a method as heating or light irradiation. According to the production method described in WO2011/115007 or WO2013/039169, the organic-inorganic composite filler (porous organic-inorganic composite filler) having an organic resin phase that covers the surface of the inorganic primary particles of the inorganic aggregated particles formed through aggregation of the inorganic primary particles and bonds the inorganic primary particles to each other, and aggregation voids formed among the organic resin phases covering the surface of the inorganic primary particles can be obtained.

While the polymerization initiator used may be a known polymerization initiator with no particular limitation, a thermal polymerization initiator is preferably used, and a thermal polymerization initiator formed of a compound having no aromatic ring in the structure thereof is more preferably used, since a cured material having a lower yellowness degree can be obtained. Examples of the thermal polymerization initiator of this type include azobisisobutyronitrile and 2,2'-azobis(2,4-dimethylvaleronitrile).

Particularly in the polymerization process and the process of pulverizing the resulting cured material, there may be cases where the orgnaic component in the organic-inorganic composite filler is discolored due to frictional heat or the like, resulting in the organic-inorganic composite filler having a high yellowness degree. The photocationically curable composition using that type of the organic-inorganic composite filler provides a cured material having a high yellowness degree. The yellowness degree of the cured material of the photocationically curable composition affects the hue of colored light confirmed from the material having colored light by interference as in the present invention.

Therefore, in the present invention, the organic-inorganic composite filler preferably has a low yellowness degree. Specifically, the organic-inorganic composite filler preferably has b* showing blue to yellow in the CIE Lab of -2.5 or less, and more preferably -3.0 or less, on the black background.

The average particle diameter of the organic-inorganic composite filler (cf) is not particularly limited, and is preferably 2 to 100 pm, more preferably 5 to 50 pm, and further preferably 5 to 30 pm, from the standpoint of the mechanical strength of the cured material of the photocationically curable composition and the improvement in handleability of the photocationically curable composition in the form of a paste. The shape thereof is not particularly limited, and examples thereof include the filler having an irregular shape obtained in such a manner that the components including the prescribed amounts of the inorganic spherical filler ($f_2$), the polymerizable monomer ($\alpha$), and the polymerization initiator, are mixed, polymerized by such a method as heating or light irradiation, and then pulverized, and the filler having a spherical or approximately spherical shape produced according to the method described in WO2011/115007 or WO2013/039169.

The organic-inorganic composite filler (cf) may contain known additives, such as a polymerization inhibitor and

a fluorescent brightening agent, in such a range that does not impair the effects thereof.

The organic-inorganic composite filler (cf) may be rinsed, or surface-treated with a silane coupling agent or the like.

The content of the organic-inorganic composite filler (cf) is preferably 50 to 1,000 parts by mass, more preferably 70 to 600 parts by mass, and further preferably 100 to 400 parts by mass, per 100 parts by mass of the cationically polymerizable monomer (a). In the case where the content of the organic-inorganic composite filler (cf) is in the range, the restoration with high color tone compatibility with the natural teeth can be easily performed, and the handleability of the paste of the photocationically curable composition and the mechanical strength of the cured material can be improved.

5-3. Radically Polymerizable Monomer

The photocationically curable composition of the present invention preferably contains a radically polymerizable monomer for the enhancement of the adhesion strength in adhering by using a dental adhesive. In the photocationically curable composition of the present invention, a radical species is formed through light irradiation, and therefore radical polymerization proceeds along with cationic polymerization. The ordinary dental adhesive is a radically polymerizable composition, and therefore a radically polymerizable monomer is preferably added in the case where the composition is used as a dental curable composition. A particularly preferred radically polymerizable monomer is a (meth)acrylic compound. With a smaller amount of the radically polymerizable monomer mixed therein, the adhesion strength to the ordinary adhesive can be enhanced while retaiing the low polymerization shrinkage rate.

Specific examples of the radically polymerizable monomer that can be preferably used include methyl (meth)acrylate, polyethylene glycol mono(meth)acrylate, 2-hydroxyethyl (meth)acrylate, ethylene glycol di(meth)acrylate, diethylene glycol di(meth)acrylate, triethylene glycol di(meth)acrylate, propylene glycol di(meth)acrylate, 1,6-hexanediol di(meth)acrylate, 1,9-nonanediol di(meth)acrylate, 2,2-bis(4-methacryloyloxyphenyl)propane, 2,2-bis(4-methacryloyloxypolyethoxyphenyl)propane, 2,2-bis[4-(2-hydroxy-3-methacryloyloxypropoxy)phenyl]propane, and trimethylolpropane trimethacrylate.

The radically polymerizable monomer may be used alone, or two or more kinds thereof used as a mixture, depending on necessity. The amount of the radically polymerizable monomer used is preferably 10 to 70 parts by mass, and more preferably 10 to 30 parts by mass, per 100 parts by mass of the cationically polymerizable monomer (a), from the standpoint that the adhesiveness can be enhanced while retaining the low polymerization shrinkage rate.

5-4. Additional Additives

In the case where the photocationically curable composition of the present invention is used as a dental composition, known additional components may be mixed therein as a composition for a dental curable composition, particularly a dental filling and restorative material, in addition to the components described above.

Examples of the components include known additives, such as an ultraviolet ray absorbent, a dye, an antistatic agent, a pigment, a perfume, an organic solvent, and a thickening agent.

The photocationically curable composition of the present invention is not limited to the dental use, but can be used as an adhesive, a paint composition, and the like, and is particularly preferred as a dental filling and restorative material.

The photocationically curable composition of the present invention that contains the inorganic spherical filler ($f_1$) and/or the organic-inorganic composite filler (cf) is preferably used as a material for restorating a tooth damaged due to caries, fracture or the like since the restoration with high color tone compatibility with the natural teeth can be performed.

The photocationically curable composition containing the inorganic spherical filler ($f_1$) and/or the organic-inorganic composite filler (cf) can restore any type of cavities in restoration of teeth with high color tone compatibility with the natural teeth. The restoration with high color tone compatibility can be performed even for a class III cavity (i.e., a cavity on a proximal surface of an incisor including no incisal angle) and a class IV cavity (i.e., a cavity on a proximal surface of an incisor including an incisal angle), to which the color tone is generally difficult to fit, making the restoration operation complicated.

6. Production Method of Photocationically Curable Composition of Present Invention

The production method of the photocationically curable composition of the present invention is not particularly limited, and a known production method of a curable composition may be appropriately used. Specifically, it suffices that the prescribed amounts of the cationically polymerizable monomer, the photo acid generator, and other components mixed depending on necessity, which constitute the curable composition of the present invention, are weighed and mixed to form a paste, in the dark. The photocationically curable composition of the present invention produced in this

manner is stored under shade.

The measure used for curing the photocationically curable composition of the present invention may be appropriately a known polymerization measure corresponding to the polymerization initiating mechanism of the photo acid generator-based polymerization initiator, and specifically, light irradiation using a light source, such as a carbon arc, a xenon lamp, a metal halide lamp, a tungsten lamp, a fluorescent lamp, solar light, helium-cadmium laser, and argon laser, heating with a heat-polymerization device or the like, a method combining these, or the like may be used with no limitation. In the case where the polymerization is performed by light irradiation, the irradiation time may be determined by performing a preliminary experiment in advance since varying depending on the wavelength and the intensity of the light source, and the shape and the material of the cured material, and in general, the mixing ratio of the components are preferably regulated to make the irradiation time within a range approximatly of 5 to 60 seconds.

## Examples

The present invention will be specifically described with reference to examples below, but the present invention is not limited to Examples.

### 1. Abbreviations of Compounds

The compounds used in Examples and Comparative Examples and the abbreviations thereof are shown below.

(1) Cationically Polymerizable Monomer (a)

- KR-470: Compound represented by the following formula (available from Shin-Etsu Chemical Co., Ltd.)
- OXT-121: Compound represented by the following formula (available from Toagosei Co., Ltd.)
- OX1: Compound represented by the following formula (available from Toagosei Co., Ltd.)
- OX2: Compound represented by the following formula (available from Ube Industries, Ltd.)

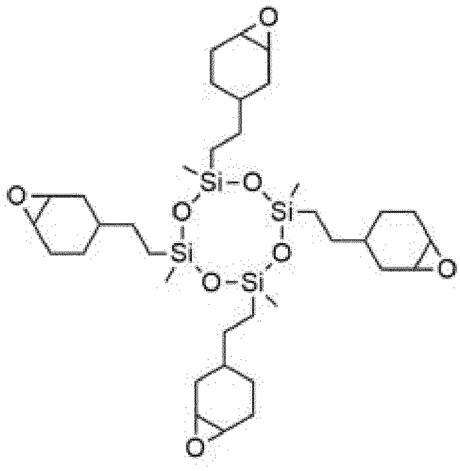

KR－470

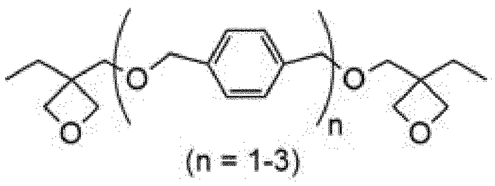

(n = 1-3)

OXT－121

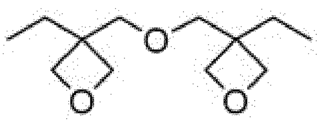

OX1

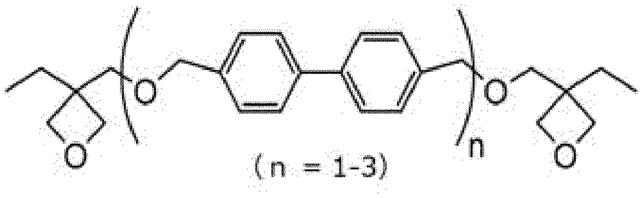

(n = 1-3)

OX2

(2) Photo Acid Generator (b)

- DPIB: Iodonium salt having tetrakispentafluorophenyl borate as counter anion represented by the following formula (available from Tokyo Chemical Industry Co., Ltd.)

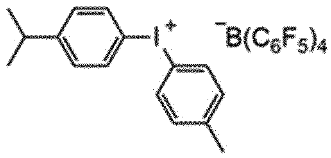

D P I B

(3) Photosensitizer (c)

- CQ: Camphorquinone (available from Tokyo Chemical Industry Co., Ltd.)

(4) Electron Donating Compound

(4-1) Thiophene Compound (d)

- Th1: 2,3,4,5-tetraphenylthiophene (available from Tokyo Chemical Industry Co., Ltd.)
- Th2: 2,2'-Bithiophene (available from Tokyo Chemical Industry Co., Ltd.)
- Th3: 5-Octyl-2,2'-bithiophene (available from Tokyo Chemical Industry Co., Ltd.)
- Th4: 5,5'-Dihexyl-2,2'-bithiophene (available from Tokyo Chemical Industry Co., Ltd.)
- Th5: 5,5"-Dibromo-2,2':5',2"-terthiophene (available from Tokyo Chemical Industry Co., Ltd.)
- Th6: 2,2'-(9,9-dioctyl-9H-fluoren-2,7-diyl)bisthiophene (available from Sigma-Aldrich Co. LLC)

The structural formulae of the thiophene compounds (d) above are shown below.

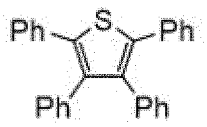

Th1

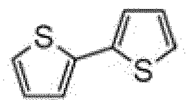

Th2

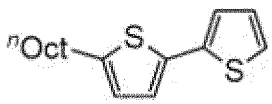

Th3

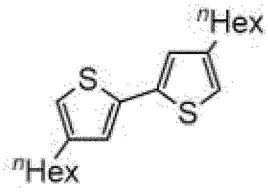

Th4

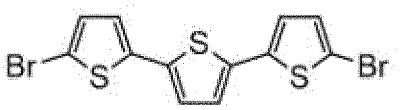

Th5

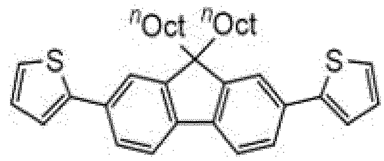

Th6

(4-2) Additional Electron Donating Compound

- DMAn: 9,10-Dimethylanthracene (available from Tokyo Chemical Industry Co., Ltd.)

- DMBE: Ethyl p-dimethylaminobenzoate (available from Tokyo Chemical Industry Co., Ltd.)
- TMBn: 1,2,4-Trimethoxybenzene (available from Sigma-Aldrich Co. LLC)
- 3Ph: p-Terphenyl (available from Tokyo Chemical Industry Co., Ltd.)

The structural formlae of the compounds above are shown below.

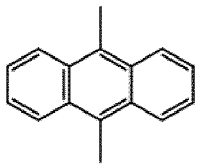

DMAn

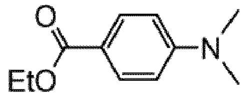

DMBE

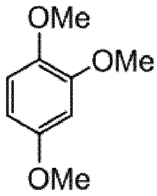

TMBn

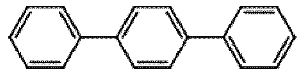

3Ph

(5) Additional Components

(5-1) Phenol-based Antioxidant

- BHT: Dibutylhydroxytoluene (available from Tokyo Chemical Industry Co., Ltd.)
- HQME: Hydroquinone monomethyl ether (available from Wako Pure Chemical Industries, Ltd.)

(5-2) Filling Material

- F1: Spherical silica-zirconia (surface-treated with γ-methacryloyloxypropyltrimethoxysilane) (5-3) Radically Polymerizable Monomer
- D-2.6E: 2,2-bis(4-Methacryloxypolyethoxyphenyl)propane (available from Shin-Nakamura Chemical Co., Ltd.)
- 3G: Triethylene glycol dimethacrylate (available from Shin-Nakamura Chemical Co., Ltd.)

2. Examples 1 to 15 and Comparative Example 1 to 13

[Exmaple 1]

1.0 part by mass of DPIB as a photo acid generator, 0.2 part by mass of CQ as a photosensitizer, 0.2 part by mass of Th1 as an electron donating compound, and 0.2 part by mass of BHT as a phenol-based antioxidant were added to 100 parts by mass of cationically polymerizable monomer containing 50 parts by mass of KR-470 and 50 parts by mass of OXT-121, and the mixture was agitated under red light for 6 hours, so as to prepare a photocationically curable composition.

The resulting photocationically curable composition was evaluated for the surface hardness of the cured material, the color tone change of the cured material, and the number of storing days until gelation (as an index of gelation resistance) by the following methods. The results are shown in Table 1.

Surface Hardness of Cured Material

The photocationically curable composition prepared was charged in a mold formed of polyacetal having a hole of 7 mm in diameter × 1.0 mm, and pressed with a polypropylene film. Subsequently, the composition was irradiated with light for 10 seconds (light intensity on irradiated surface: 400 $mW/cm^2$) with a dental light irradiator (Tokuso Power Light, available from Tokuyama Dental Corporation). Immediately after the light irradiation, the state of the charged material was confirmed as to whether the entire composition was cured. In the case where the entire composition was cured, the resulting cured material was taken out from the mold, and with a micro-hardness meter (Model MMT-X7, available from Matsuzawa Seiki Co., Ltd.), the diagonal length d of the dent formed on the test piece with a Vickers indenter under a load of 100 gf for a load retention time of 30 seconds was measured, from which the surface hardness Hv of the test piece was obtained. The surface hardness Hv was obtained according to the following expression (1).

$$Hv = 1854.37 \times 100 / d^2 \qquad (1)$$

Color Tone Change of Cured Material

A cured material was prepared in the same manner as in the evaluation of the surface hardness of the cured material, and immediately after the light irradiation, measured for the color tone with a spectral colorimeter (SE7700, available from Nippon Denshoku Industries Co., Ltd.). The cured material after measuring the color tone was stored under shading condition for 7 days, and then again measured for the color tone in the same manner. The color difference ΔE* between immediately after the light irradiation and after storing for 7 days was designated as the value of color tone change.

The evaluation standard of the color tone change by ΔE* is as follows.

$0.8 \leq \Delta E^* < 1.6$: allowable tolerance class AA (only slight color difference perceived)
$1.6 \leq \Delta E^* < 3.2$: allowable tolerance class A (color difference substantially not detected)
$3.2 \leq \Delta E^* < 6.5$: allowable tolerance class B (same color under impression)
$6.5 \leq \Delta E^* < 13.0$: allowable tolerance class C (color difference corresponding to one step in JIS Color Chart, Munsell Color Chart, etc.)

Gelation Time

0.1 g of the photocationically curable composition prepared was placed in a 2 mL screw tube, and stored in a thermostat chamber at 50°C under shading condition. The photocationically curable composition was taken out from the thermostat chamber every other day, and after allowing to cool to room temperature, the state of the curable composition was observed. At this time, the number of days until the photocationically curable composition was gelled, i.e., completely did not flow in tilting the sample tube, was designated as the gelation time.

[Examples 2 to 5 and Comparative Examples 1 to 5]

Photocationically curable compositions were prepared in the same manner as in Example 1 except that the electron donating compound mixed therein was changed as shown in Table 1, and evaluated for the surface hardness of the cured material, the color tone change of the cured material, and the number of storing days until gelation, in the same manner as in Exmaple 1. The results are shown in Table 1.

Table 1

| | Electron donating compound | Surface hardness (Hv) | Color tone change (ΔE*) | Gelation time (day) |
|---|---|---|---|---|
| | | | White background | |
| Example 1 | Th1 (0.2) | 9.6 | 1.5 | 19 |
| Example 2 | Th2 (0.2) | 10.5 | 1.5 | 20 |
| Example 3 | Th3 (0.2) | 10.0 | 1.8 | 20 |
| Example 4 | Th4 (0.2) | 11.0 | 1.9 | 20 |
| Example 5 | Th5 (0.2) | 12.4 | 2.5 | 20 |
| Comparative Example 1 | DMAn (0.2) | 11.6 | 9.9 | 19 |
| Comparative Example 2 | DMBE (0.2) | 9.0 | 6.3 | 20 |
| Comparative Example 3 | TMBn (0.2) | curing failure | unmeasurable | 20 |
| Comparative Example 4 | 3Ph (0.2) | curing failure | unmeasurable | 19 |

(continued)

| | Electron donating compound | Surface hardness (Hv) | Color tone change (ΔE*) | Gelation time (day) |
|---|---|---|---|---|
| | | | White background | |
| Comparative Example 5 | - | curing failure | unmeasurable | 19 |
| The values in the table are the addition amounts part by mass) of the components.<br>- Polymerizable monomer: KR-470 (50 parts by mass), OXT-121 (50 parts by mass)<br>- Photo acid generator: DPIB (1.0 part by mass)<br>- Photosensitizer: CQ (0.2 part by mass)<br>- Antioxidant: BHT (0.2 part by mass) | | | | |

As shown in Table 1 above, Exmaples 1 to 5 using the thiophene compound as the electron donating compound each had a high surface hardness (9 Hv or more) of the cured material, and the allowable tolerance class A for the color tone stability evaluation. On the other hand, Compartive Examples 1 and 2 using the anthracene compound DMAn or the aroamtic amine compound DMBE as the electron donating compound had good hardenability, but the color tone stability evaluation thereof was poor, i.e., the allowable tolerance class C for Comparative Example 1 and the allowable tolerance class B for Comparative Example 2. Comparative Examples 3 and 4 using TMBn or 3Ph as the electron donating compound and Comparative Example 5 using no electron donating compound were not able to prepare a cured material since the entire specimen was not cured after the light irradiation. The gelation time was approximately 20 days for all the specimens in Table 1, and there was no significant difference found.

[Examples 6 to 11 and Comparative Examples 6 to 9]

Compositions were prepared in the same manner as in Example 1 except that the formulation of the cationically polymerizable monomer was changed to KR-470 (60 parts by mass), OX1 (26 parts by mass), and OX2 (14 parts by mass), 7 parts by mass and 4 parts by mass (11 parts by mass in total) of D-2.6E and 3G, respectively, were added as radically polymerizable monomers to 100 parts by mass of the cationically polymerizable monomer, and the kinds and the amounts of the electron donating compound and other additives further mixed therein were changed as shown in Table 2, and evaluated for the surface hardness of the cured material, the color tone change of the cured material, and the number of storing days until gelation, in the same manner as in Exmaple 1. The results are shown in Table 2.

Table 2

| | Electron donating compound | Surface hardness (Hv) | Color tone change (ΔE*) | Gelation time (day) |
|---|---|---|---|---|
| | | | White background | |
| Example 6 | Th3 (0.1) | 10.5 | 1.6 | 18 |
| Example 7 | Th3 (0.6) | 10.9 | 3.0 | 21 |
| Example 8 | Th4 (0.1) | 9.6 | 0.9 | 18 |
| Example 9 | Th4 (0.6) | 9.6 | 0.9 | 18 |
| Example 10 | Th6 (0.1) | 10.6 | 2.2 | 18 |
| Example 11 | Th6 (0.6) | 11.7 | 3.1 | 18 |
| Comparative Example 6 | DMAn (0.1) | 10.1 | 6.4 | 15 |
| Comparative Example 7 | DMAn (0.6) | 11.2 | 5.9 | 18 |
| Comparative Example 8 | DMBE (0.1) | 7.7 | 2.7 | 15 |

(continued)

| | Electron donating compound | Surface hardness (Hv) | Color tone change (ΔE*) | Gelation time (day) |
|---|---|---|---|---|
| | | | White background | |
| Comparative Example 9 | DMBE (0.6) | 8.4 | 7.0 | 18 |
| The values in the table are the addition amounts part by mass) of the components.<br>- Polymerizable monomer: KR-470 (60 parts by mass), OX1 (26 parts by mass), OX2 (14 parts by mass)<br>- Photo acid generator: DPIB (2.0 part by mass)<br>- Photosensitizer: CQ (0.3 part by mass)<br>- Antioxidant: HQME (0.1 part by mass), BHT (0.1 part by mass)<br>- Ultraviolet ray absorbent: SS701 (0.1 part by mass)<br>- Radically polymerizable monomer: D-2.6E (7 parts by mass), 3G (4 parts by mass) | | | | |

Examples 6 to 11 were examples using the photocationically curable composition of the present invention, in which the formulation of the cationically polymerizable monomer was slightly changed, and the electron donating compound and the thiophene compound were added with the kinds and amounts thereof changed, to the polymerizable monomer composition having the prescribed amount of the radically polymerizable monomer mixed therein, and exhibited favorable curability, color tone stability, and gelation resistance, as similar to Exmaples 1 to 5 containing no radically polymerizable monomer. On the other hand, Comparative Exmaples 6 to 9 having DMAn or DMBE mixed in the same polymerizable monomer composition had the same tendency as in Comparative Examples 1 and 2 containing no radically polymerizable monomer, and Comparative Examples 6 and 8 showed a tendency that the gelation resistance was slightly lowered.

[Examples 12 to 15 and Comparative Examples 10 to 13]

Compositions were prepared in the same manner as in Example 6 except that the kind and the amount of the radically polymerizable monomer, the amount of the filling material, the kind and the amount of the electron donating compound, and the amounts of tother catalysts (i.e., the photo acid generator and the sensitizer) mixed in 100 parts by mass of the cationically polymerizable monomer were changed as shown in Table 3, and evaluated for the surface hardness of the cured material and the color tone change of the cured material, in the same manner as in Exmaple 1. The the number of storing days until gelation was measured in the same manner as in Example 1 except that the storing temperature was changed from 50°C to 37°C for evaluating at a temperature close to the actual storing condition, and the adhesion strength and the polymerization shrinkage rate were evaluated by the following methods. The results are shown in Table 3.

Adhesion Strength

A dental resin block cured material was polished with a polisher using waterproof abrasive paper #120 and then #600, and then subjected to a sandblast treatment. On the surface subjected to the sandblast treatment, a double-sided adhesive tape having a thickness of 180 pm having a hole having a diameter of 3 mm was fixed, and then paraffin wax having a thickness of 0.5 mm having a hole having a diameter of 8 mm was fixed concentrically to the circular hole, so as to form a simulated caivity. A dental adhesive (Bondmer Lightless, available from Tokuyama Dental Corporation) was coated in the simulated cavity, and after allowing to stand for 10 seconds, dried by blowing compressed air for 10 seconds. The photocationically curable composition prepared was coated further thereon, pressed with a polypropylene film, and irradiated with light for 20 seconds (light intensity on irradiated surface: 800 mW/$cm^2$) with a dental light irradiator (Tokuso Power Light, available from Tokuyama Dental Corporation), so as to produce an adhesion test piece. Thereafter, a metal attachment was adhered to the cured material with a dental cement. The assembly was immersed in water at 37°C for 24 hours, and then pulled with a tensile tester (Autograph, available from Shimadzu Corporation) at a crosshead speed of 2 mm/min, so as to measure the tensile adhesion strength of the adherend (i.e., the dental resin block cured material) and the photocationically curable composition. Four specimens per one test were measured for the tensile adhesion strength by the aforementioned method, and the average value was designated as the value of the adhesion strength.

Polymerization Shrinkage Rate

A stainless steel plunger having a diameter of 3 mm and a height of 4 mm was inserted to a stainless steel split mold having a diameter of 3 mm and a height of 7 mm, and the height of the hole was set to 3 mm. Subsequently, the photocationically curable composition prepared was charged in the hole, and the upper end of the hole was pressed with a polypropylene film. Thereafter, the surface of the stainless steel split mold having the polypropylene film attached thereto was directed downward, and in this state, placed on the glass table of a dental light irradiator (Tokuso Power Light, available from Tokuyama Dental Corporation). A probe capable of measuring a minute movement of the probe was brought into contact therewith from above the stainless steel plunger. In this state, the dental curable composition was polymerized and cured with the dental irradiator (light intensity on irradiated surface: 800 $mW/cm^2$), and the shrinkage rate (%) after 3 minutes from the start of irradiation was calculated from the moving distance of the probe in the vertical direction.

Table 3

| | Cationically polymerizable monomer | Radically polymerizable monomer | Electron donating compound | Other catalysts | | Filler | Surface hardness (Hv) | Color tone change ($\Delta E^*$) | Gelation time (day) | Adhesion strength (MPa) | Shrinkage rate (%) |
|---|---|---|---|---|---|---|---|---|---|---|---|
| | | | | Photo acid generator | Photosensitizer | | | White background | | | |
| Example 12 | KR-470 (50), OX1 (50) | - | Th3 (0.5) | DPIB (2.0) | CQ (0.3) | F1 (250) | 21.0 | 3.0 | 21 | 8.5 | 1.4 |
| Example 13 | KR-470 (60), OX1 (26), OX2 (14) | D-2.6E (7), 3G (4) | Th3 (0.6) | DPIB (2.2) | CQ (0.3) | F1 (280) | 21.7 | 3.1 | > 30 | 15.4 | 1.7 |
| Example 14 | KR-470 (63), OX1 (25), OX2 (12) | D-2.6E (15), 3G (10) | Th3 (0.6) | DPIB (2.5) | CQ (0.4) | F1 (313) | 22.5 | 2.8 | > 30 | 17.0 | 1.9 |
| Example 15 | KR-470 (67), OX1 (25), OX2 (8) | D-2.6E (40), 3G (27) | Th3 (0.8) | DPIB (3.3) | CQ (0.5) | F1 (418) | 23.7 | 2.6 | > 30 | 25.4 | 2.0 |
| Comparative Example 10 | KR-470 (50), OX1 (50) | - | DMAn (0.5) | DPIB (2.0) | CQ (0.3) | F1 (250) | 22.2 | 4.5 | 10 | 8.6 | 1.5 |
| Comparative Example 11 | KR-470 (60), OX1 (26), OX2 (14) | D-2.6E (7), 3G (4) | DMAn (0.6) | DPIB (2.2) | CQ (0.3) | F1 (280) | 23.4 | 3.9 | > 30 | 17.7 | 1.8 |
| Comparative Example 12 | KR-470 (50), OX1 (50) | - | DMBE (0.5) | DPIB (2.0) | CQ (0.3) | F1 (250) | 6.6 | 9.2 | 20 | 9.2 | 1.0 |
| Comparative Example 13 | KR-470 (60), OX1 (26), OX2 (14) | D-2.6E (7), 3G (4) | DMBE (0.6) | DPIB (2.2) | CQ (0.3) | F1 (280) | 7.3 | 8.6 | 24 | 15.8 | 1.2 |

The values in the table are the addition amounts (part by mass) of the components.
- Antioxidant: HQME (0.1 part by mass), BHT (0.1 part by mass)
- Ultraviolet ray absorbent: SS701 (0.1 part by mass)

The same tendencies as described above were confirmed in the results of the surface strength and the color tone change in Exmaple 12 and Comparative Exmaples 10 and 12, and Example 13 and Comparative Examples 11 and 13, each combination of which had the same formulation except for the electron donating compound. It was also confirmed from the results of Examples 12 to 15, which differed from each other mainly in the amount of the radically polymerizable monomer within Examples, that there was a tendency that by increasing the amount of the radically polymerizable monomer mixed therein, the adhesion strength and the gelation resistance were enhanced, but the shrinkage rate was increased. However, Example 15 having the largest amount of the radically polymerizable monomer exhibited a polymerization shrinkage rate of 2.0%, which was significantly lower than the polymerization shrinkage rate of 3.2% in the case where no cationically polymerizable monomer was contained (i.e., the polymerizable monomer containing only the radically polymerizable monomer was used).

3. Examples 16 to 33 and Comparative Example 14 to 19

Photocationically curable compositions of Examples and Comparative Examples were produced and evaluated by using the inorganic spherical filler ($f_1$) and the organic-inorganic composite filler (cf) shown below.

<Inorganic Spherical Filler ($f_1$) and Inorganic Spherical Filler ($f_2$)>

The inorganic spherical fillers ($f_1$) and the inorganic spherical fillers ($f_2$) used for producing the organic-inorganic composite fillers (cf) were as follows.

Table 4

| Name of filler | Composition | Shape | Average primary particle diameter (nm) | Refractive index | Average evenness degree | Proportion of particles existing within range above and below 5% of average primary particle diameter with respect to total number of particles |
|---|---|---|---|---|---|---|
| F1 | silica-zirconia | spherical | 260 | 1.522 | 0.90 | 93 |
| F2 | silica-zirconia | spherical | 280 | 1.522 | 0.92 | 93 |
| F3 | silica-zirconia | spherical | 230 | 1.515 | 0.90 | 92 |
| F4 | silica-zirconia | spherical | 230 | 1.522 | 0.90 | 93 |

The inorganic spherical fillers (F1 to F4) were prepared by the method described in JPS58-110414A, JPS58-156524A, and the like, i.e., the so-called sol-gel method, in which a mixed solution containing a hydrolyzable organic silicon compound (such as tetraethyl silicate) and a hydrolyzable organic titanium compound (such as tetrabutyl zirconate) was added to an ammoniacal alcohol (such as methanol, ethanol, isopropyl alcohol, and isobutyl alcohol) solution having aqueous ammonia introduced thereto, so that hydrolysis is performed to deposit the reaction product.

The fillers F1, F2, and F3 were surface-treated with a silane coupling agent represented by S-1 below. The filler F4 was surface-treated with a silane coupling agent represented by S-2 below.

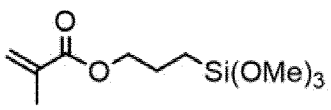

S- 1

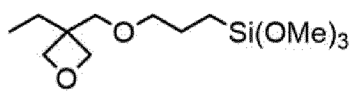

S- 2

<Organic-Inorganic Composite Filler (cf)>

In the production of the organic-inorganic composite fillers CF1 to CF4, "D-2.6E" and "HD" as the radically polymerizable monomers and "OXE-30" as the cationically polymerizable monomer were used. The following "V65" was

used as a thermal polymerization initiator.

Radically Polymerizable Monomers

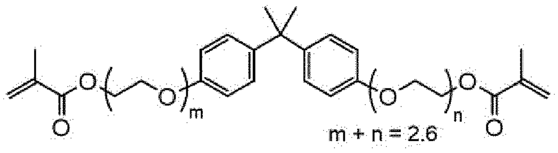

D−2. 6E

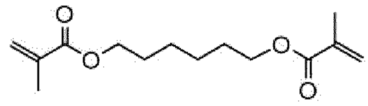

HD

Cationically Polymerizable Monomer

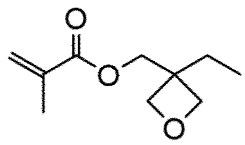

OXE−30

Thermal Polymerization Initiator

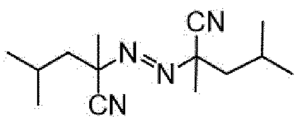

V65

(Production of CF1)

A radically polymerizable monomer containing 8 parts by mass of D-2.6E and 92 parts by mass of HD was prepred, in which V65 was dissolved to make a content of 0.02% by mass, and 300 parts by mass of F1 as the inorganic spherical filler ($f_2$) was added, followed by forming into a paste with a mortar. The paste was polymerized and cured by heating at 95°C in a nitrogen atmosphere for 1 hour. The cured material was pulverized with a vibration ball mill, so as to provide the organic-inorganic composite filler CF1.

(Production of CF2 to CF4)

The organic-inorganic composite fillers CF2 to CF4 were produced in the same manner as in CF1 except that the kind and the amount of the polymerizable monomer and the kind of the inorganic spherical filler were changed as shown in Table 5. The values in the parentheses each are in terms of part by mass. The average particle diameters shown in Table 5 each are the average particle diameter of the resulting organic-inorganic composite filler.

Table 5

| | Polymerizable monomer | Inorganic spherical filler | Content of inorganic spherical filler (% by mas) | Average particle diameter (μm) |
|---|---|---|---|---|
| CF1 | D-2.6E (8)<br>HD (92) | F1 (300) | 75 | 27 |
| CF2 | D-2.6E (8)<br>HD (92) | F2 (300) | 75 | 26 |
| CF3 | HD (100) | F1 (300) | 75 | 24 |
| CF4 | HD (80)<br>OXE-30 (20) | F1 (300) | 75 | 28 |

The structures of KR-470 and OXT-121 as the cationically polymerizable monomers, DPIB as the photo acid generator, CQ as the photosensitizer, Th2, Th3, DMBE, and DMAn as the electron donating compounds, and BHT and HQME as the phenol-based antioxidants used in Examples 16 to 33 and Comparative Examples 14 to 19 are as described above.

The radically polymerizable monomers used in Comparative Examples 16 and 19 were as follows.

- UDMA: 1,6-bis(Methacrylethyloxycarbonylamino)trimethylhexane
- 3G: Triethylene glycol dimethacrylate

The structural formulae of the compounds are shown below.

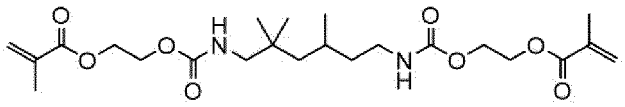

UDMA

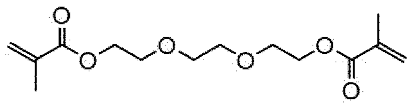

3G

The measurement methods in Examples 16 to 33 and Comparative Examples 14 to 19 were as follows. The surface hardness, the color tone change, the gelation time, and the polymerization shrinkage rate were evaluated in the same methods as in Examples 1 to 15 described above.

(1) Average Primary Particle Diameter of Inorganic Spherical Filler

A photograph of the powder was taken with a scanning electron microscope ("XL-30S", available from Koninklijke Philips N.V.), the number of all the particles (30 particles or more) observed within the unit view field of the photograph and the primary particle diameters (maximum diameters) of all the particles were measured, and the average primary particle diameter was calculated from the measured values according to the following expression.

$$\bar{X} = \frac{\sum_{i=1}^{n} X_i}{n} \text{ (number average)}$$

wherein n represents the number of the particles, and $X_i$ represents the primary particle diameter (maximum diameter) of the i-th particle.

(2) Average Evenness Degree of Inorganic Spherical Filler

A photograph of the powder was taken with a scanning electron microscope, and the particles (30 particles or more) observed within the unit view field of the photograph each were measured for the long diameter (Li) as the maximum diameter and the short diameter (Bi) as the diameter in the direction perpendicular to the long diameter, from which the average evenness degree was calculated according to the following expression.

$$\text{Average evenness degree} = \frac{\sum_{i=1}^{n} Bi/Li}{n}$$

wherein n represents the number of the particles, Bi represents the short diameter of the i-th particle, and Li represents the long diameter of the i-th particle.

(3) Proportion of Particles existing within Range of ±5% of Average Primary Particle Diameter with respect to Total Number of Particles

In all the particles (30 particles or more) in the unit view field of the photograph taken in the item (1) above, the number of particles having a primary particle diameter (maximum diameter) outside a range of ±5% of the average primary particle diameter obtained in the item (1) above was measured, and the resulting value was subtracted from the number of all the particles to provide the number of particles within a range of ±5% of the average primary particle diameter within the unit view field of the photograph, from which the proportion was calculated according to the following expression.

Proportion (%) of particles existing within range above and below 5% of average primary particle diameter with respect to number of all particles = [(number of particles existing within range above and below 5% of average primary particle diameter in the unit view field of a photograph taken by a scanning electron microscope)/(number of all particles in the unit view field of a photograph taken by a scanning electron microscope)] × 100

(4) Refractive Index nP of Polymer of Polymerizable Monomer

The refractive index of the polymer of the polymerizable monomer was obtained by measuring a polymer polymerized under the substantially same condition as the polymerization condition in the cavity with an Abbe refractometer (available from Atago Co., Ltd., light source wavelength: 589 nm) in a thermostat chamber at 25°C. The polymerizable monomer was the mixture of 75 parts by mass of KR-470 and 25 parts by mass of OXT-121 in Examples 16 to 33, Comparative Examples 14 and 15, and Comparative Examples 17 and 18, and the mixture of 80 parts by mass of UDMA and 20 parts by mass of 3G in Comparative Example 16 and Comparative Example 19.

100 parts by mass of the polymerizable monomer used in each of Examples and Comparative Examples, 2 parts by mass of DPIB as the photo acid generator, 0.3 part by mass of CQ as the photosensitizer, and 0.5 part by mass of DMAn as the electron donating compound were mixed to prepare a composition. The composition was held by two polypropylene films to make the thickness of the composition approximately of 0.02 to 0.1 mm. Light irradiation was then performed for 10 seconds with a dental light irradiator (Tokuso Power Light, available from Tokuyama Dental Corporation). The light irradiator radiated light having a wavelength range of 400 to 500 nm. The irradiaiton was performed to make the light intensity on the irradiated surface of 400 $mW/cm^2$. After the light irradiation, the polymer of the polymerizable monomer in the form of a film was released from the polypropylene films. In mounting the polymer on an Abbe refractometer (available from Atago Co., Ltd., light source wavelength: 589 nm), a solvent (bromonaphthalene) that did not dissolve the specimen and had a higher refractive index than the specimen was dropped on the specimen for the purpose of bringing the polymer into close contact with the measuring surface, and then the specimen was measured.

(5) Refractive Indices ($nF_a$ and $nF_b$) of Inorganic Spherical Fillers

The refractive index of the inorganic spherical filler used was measured with an Abbe refractometer (available from Atago Co., Ltd., light source wavelength: 589 nm) by the immersion method.

Specifically, in a thermostat chamber at 25°C, 1 g of the inorganic spherical filler was dispersed in 50 mL of anhydrous toluene in a 100 mL sample tube. 1-Bromotoluene was dropped gradually on the dispersion liquid under stirring with a stirrer, and the refractive index of the dispersion liquid at which the dispersion liquid became most transparent was measured to measure the refractive index of the inorganic spherical filler.

(6) Refractive Index (nM) of Organic Resin Matrix

The refractive index nM of the organic resin matrix was obtained by measuring a polymer polymerized under the substantially same condition as the polymerization condition in the production of the organic-inorganic composite filler with an Abbe refractometer (available from Atago Co., Ltd., light source wavelength: 589 nm) in a thermostat

chamber at 25°C.

Specifically, V65 was added to and mixed with the polymerizable monomer used for producing the organic resin matrix to make the content thereof of 0.02% by mass, and the uniform polymerizable monomer (or the uniform mixture of polymerizable monomers) was held by two polypropylene films to make the thickness of the composition approximately of 0.02 to 0.1 mm. Thereafter, the composition was polymerized and cured by heating at 95°C under pressurization with nitrogen for 1 hour, and the polymer was relased from the polypropylene films to produce a polymer in the form of a film (organic resin matrix) of polymerizable monomers. In mounting the polymer on an Abbe refractometer (available from Atago Co., Ltd., light source wavelength: 589 nm), a solvent (bromonaphthalene) that did not dissolve the specimen and had a higher refractive index than the specimen was dropped on the specimen for the purpose of bringing the polymer into close contact with the measuring surface, and then the refractive index was measured.

(7) Visual Evaluation of Colored Light

The paste of the photocationically curable composition prepared in each of Exmaples and Comparative Examples was placed in a mold having a hole of 7 mm in diameter × 1 mm, and pressed with polypropylene films from both sides. The composition was cured through light irradiation on both surfaces for 30 seconds for each with a dental light irradiator (Tokuso Power Light, available from Tokuyama Dental Corporation), and then the cured material taken out from the mold was placed on an adhesive surface of a black adhesive tape (carbon tape) in the form of an approximately 10 mm square, and visually confirmed for the color tone of the colored light.

(8) Visual Evaluation of Fluorescent Light

The paste of the photocationically curable composition prepared in each of Exmaples and Comparative Examples was placed in a mold having a through hole of 7 mm in diameter × 1 mm, and pressed with polypropylene films from both sides. The composition was cured through light irradiation on both surfaces for 30 seconds for each with a dental light irradiator (Tokuso Power Light, available from Tokuyama Dental Corporation), and then the cured material was taken out from the mold. The cured material was irradiated with ultraviolet light (wavelength: 365 nm) with an ultraiviolet light lamp (Handy UV Lamp, available from Spectronix Corporation), and confirmed as to whether particular coloration occurred as compared to the natural teeth.

(9) Spectral Reflectance Ratio

The paste of the photocationically curable composition prepared in each of Exmaples and Comparative Examples was placed in a mold having a through hole of 7 mm in diameter × 1 mm, and pressed with polypropylene films from both sides. The composition was cured through light irradiation on both surfaces for 30 seconds for each with a dental light irradiator (Tokuso Power Light, available from Tokuyama Dental Corporation). The cured material was taken out from the mold and measured for the spectral reflectance ratio on black background with a colorimeter ("TC-1800MKII", available from Tokyo Denshoku Co., Ltd.), from which the maximum value of the reflectance SR1 in the yellow to red region (600 to 750 nm) and the maximum value of the reflectance SR2 in the blue region (400 to 500 nm) were obtained, and the spectral reflectance ratio was calculated by dividing SR1 by SR2.

With a spectral reflectance ratio (SR1/SR2) of 0.8 to 2.0, the yellowish to reddish colored light is more intense than the blue colored light, which facilitates the restoration with high color tone compatibility with the natural teeth.

[Example 16]

2.0 parts by mass of DPIB as the photo acid generator, 0.3 part by mass of CQ as the photosensitizer, 0.1 part by mass of Th3 as the electron donating compound, 150 parts by mass of F1 as the inorganic spherical filler, and 0.1 part by mass of HQME and 0.1 part by mass of BHT as the phenol-based antioxidants were added to 100 parts by mass of the cationically polymerizable monomer containing 75 parts by mass of KR-470 and 25 parts by mass of OXT-121, and the mixture was agitated under red light for 6 hours, so as to prepare a photocationically curable composition. The resulting photocationically curable composition was evaluated by the aforementined methods. The results are shown in Table 6.

[Examples 17 to 24 and Comparative Examples 14 and 15]

Photocationically curable compositions were prepared in the same manner as in Example 16 except that the kinds and the amounts of the electron donating compound and the inorganic spherical filler were changed as shown in Table 6. The resulting photocationically curable compositions were evaluated by the aforementined methods. The results

are shown in Table 6.

[Comparative Example 16]

A photocationically curable composition was prepared in the same manner as in Example 16 except that a radically polymerizable monomer containing 80 parts by mass of UDMA and 20 parts by mass of 3G was used instead of the cationically polymerizable monomer, and the amount of the electron donating compound mixed was changed to 0.5 part by mass. The resulting photocationically curable composition was evaluated by the aforementined methods. The results are shown in Table 6.

Table 6

| | Cationically polymerizable monomer | Radically polymerizable monomer | Electron donating compound | Inorganic spherical filler | Surface hardness (Hv) | Color tone change (ΔE*) white background | Gelation time (day) | Polymerization shrinkage rate (%) | Visual evaluation of colored light | Visual evaluation of fluorescent light | Spectral reflectance ratio |
|---|---|---|---|---|---|---|---|---|---|---|---|
| Example 16 | KR-470 (75) | | Th3 (0.1) | F1 (150) | 20.8 | 2.9 | 27 | 1.3 | yellow | - | 1.09 |
| | UXT-121 (25) | | | | | | | | | | |
| | nP = 1.509 | | | $nF_a$ = 1.522 | | | | | | | |
| Example 17 | KR-470 (75) | | Th3 (0.3) | F1 (150) | 22.3 | 2.8 | > 30 | 1.4 | yellow | - | 1.12 |
| | UXT-121 (25) | | | | | | | | | | |
| | nP = 1.509 | | | $nF_a$ = 1.522 | | | | | | | |
| Example 18 | KR-470 (75) | | Th3 (0.5) | F1 (150) | 22.5 | 3.3 | > 30 | 1.4 | yellow | - | 1.25 |
| | UXT-121 (25) | | | | | | | | | | |
| | nP = 1.509 | | | $nF_a$ = 1.522 | | | | | | | |
| Example 19 | KR-470 (75) | | Th3 (0.5) | F2 (150) | 22.2 | 3.3 | > 30 | 1.5 | red | - | 1.08 |
| | UXT-121 (25) | | | | | | | | | | |
| | nP = 1.509 | | | $nF_a$ = 1.522 | | | | | | | |
| Example 20 | KR-470 (75) | | Th3 (0.5) | F3 (150) | 20.6 | 3.5 | > 30 | 1.4 | yellow | - | 0.95 |
| | UXT-121 (25) | | | | | | | | | | |
| | nP = 1.509 | | | $nF_a$ = 1.515 | | | | | | | |
| Example 21 | KR-470 (75) | | Th3 (0.5) | F4 (150) | 24.9 | 2.9 | > 30 | 1.4 | yellow | - | 1.18 |
| | UXT-121 (25) | | | | | | | | | | |
| | nP = 1.509 | | | $nF_a$ = 1.522 | | | | | | | |

(continued)

| | Cationically polymerizable monomer | Radically polymerizable monomer | Electron donating compound | Inorganic spherical filler | Surface hardness (Hv) | Color tone change ($\Delta E^*$) white background | Gelation time (day) | Polymerization shrinkage rate (%) | Visual evaluation of colored light | Visual evaluation of fluorescent light | Spectral reflectance ratio |
|---|---|---|---|---|---|---|---|---|---|---|---|
| Example 22 | KR-470 (75) | | Th3 (0.5) | F1 (100) | 20.9 | 3.6 | > 30 | 1.8 | yellow | - | 0.93 |
| | UXT-121 (25) | | | | | | | | | | |
| | nP = 1.509 | | | $nF_a$ = 1.522 | | | | | | | |
| Example 23 | KR-470 (75) | | Th3 (0.5) | F1 (200) | 24.5 | 3.1 | 27 | 1.2 | yellow | - | 1.20 |
| | UXT-121 (25) | | | | | | | | | | |
| | nP = 1.509 | | | $nF_a$ = 1.522 | | | | | | | |
| Example 24 | KR-470 (75) | | Th2 (0.5) | F1 (150) | 20.5 | 3.5 | > 30 | 1.4 | yellow | - | 1.19 |
| | UXT-121 (25) | | | | | | | | | | |
| | nP = 1.509 | | | $nF_a$ = 1.522 | | | | | | | |
| Comparative Example 14 | KR-470 (75) | | DMBE (0.5) | F1 (150) | 7.8 | 9.5 | > 30 | 1.4 | yellow | - | 1.08 |
| | UXT-121 (25) | | | | | | | | | | |
| | nP = 1.509 | | | $nF_a$ = 1.522 | | | | | | | |
| Comparative Example 15 | KR-470 (75) | | DMAn (0.5) | F1 (150) | 22.1 | 4.3 | 21 | 1.4 | yellow | blue | 0.32 |
| | UXT-121 (25) | | | | | | | | | | |
| | nP = 1.509 | | | $nF_a$ = 1.522 | | | | | | | |

(continued)

| | Cationically polymerizable monomer | Radically polymerizable monomer | Electron donating compound | Inorganic spherical filler | Surface hardness (Hv) | Color tone change ($\Delta E^*$) white background | Gelation time (day) | Polymerization shrinkage rate (%) | Visual evaluation of colored light | Visual evaluation of fluorescent light | Spectral reflectance ratio |
|---|---|---|---|---|---|---|---|---|---|---|---|
| Comparative Example 16 | | UDMA (80) | Th3 (0.5) | F1 (150) | 16.3 | 3.1 | > 30 | 3.0 | yellow | - | 1.05 |
| | | 3G (20) | | | | | | | | | |
| | | nP = 1.509 | | $nF_a$ = 1.522 | | | | | | | |
| The values in parentheses show the amounts (part by mass) of the components added.<br>nP shows the refractive index (25°C) of the polymer of the polymerizable monomer, and $nF_a$ shows the refractive index (25°C) of the inorganic spherical filler.<br>Additional components: DPIB (2.0), CQ (0.3), HQME (0.1), BHT (0.1) | | | | | | | | | | | |

It was understood that the photocationically curable compositions of Examples 16 to 24 had strong yellow to red colored light, and were capable of performing restoration with high color tone compatibility with the natural teeth. Furthermore, the photocationically curable compositions of the present invention had good properties, i.e., small polymerization shrinkage and a high surface hardness of the cured material.

On the other hand, the photocationically curable composition of Comparative Example 14 using the aromatic amine as the electron donating compound, but not using the thiophene compound, had a decreased surface hardness of the cured material.

It was understood that the photocationically curable composition of Comparative Example 15 using the anthracene compound as the electron donating compound, but not using the thiophene compound, was confirmed to show blue fluorescent light under ultraviolet light, and also had a low spectral reflectance ratio, resulting in difficulty in performing restoration with high color tone compatibility.

The photocationically curable composition of Comparative Example 16 using the radically polymerizable monomer instead of the cationically polymerizable monomer resulted in a larger polymerization shrinkage rate than Examples.

[Exmaple 25]

2.0 parts by mass of DPIB as the photo acid generator, 0.3 part by mass of CQ as the photosensitizer, 0.1 part by mass of Th3 as the electron donating compound (thiophene compound), 200 parts by mass of CF1 as the organic-inorganic composite filler, and 0.1 part by mass of HQME and 0.1 part by mass of BHT as the phenol-based antioxidants were added to 100 parts by mass of the cationically polymerizable monomer containing 75 parts by mass of KR-470 and 25 parts by mass of OXT-121, and the mixture was agitated under red light for 6 hours, so as to prepare a photocationically curable composition. The resulting photocationically curable composition was evaluated by the aforementined methods. The results are shown in Table 7.

[Examples 26 to 31 and Comparative Examples 17 and 18]

Photocationically curable compositions were prepared in the same manner as in Example 25 except that the kinds and the amounts of the electron donating compound and the organic-inorganic composite filler were changed as shown in Table 7. The resulting photocationically curable compositions were evaluated by the aforementined methods. The results are shown in Table 7.

[Example 32]

2.0 parts by mass of DPIB as the photo acid generator, 0.3 part by mass of CQ as the photosensitizer, 0.5 part by mass of Th3 as the electron donating compound (thiophene compound), 100 parts by mass of CF1 as the organic-inorganic composite filler, 100 parts by mass of F1 as the inorganic spherical filler, and 0.1 part by mass of HQME and 0.1 part by mass of BHT as the phenol-based antioxidants were added to 100 parts by mass of the cationically polymerizable monomer containing 75 parts by mass of KR-470 and 25 parts by mass of OXT-121, and the mixture was agitated under red light for 6 hours, so as to prepare a photocationically curable composition. The resulting photocationically curable composition was evaluated by the aforementined methods. The results are shown in Table 7.

[Example 33]

A photocationically curable composition was prepared in the same manner as in Example 32 except that the amounts of the organic-inorganic composite filler and the inorganic spherical filler were changed as shown in Table 7. The resulting photocationically curable composition was evaluated by the aforementined methods. The results are shown in Table 7.

[Comparative Exmaple 19]

A photocationically curable composition was prepared in the same manner as in Example 25 except that a radically polymerizable monomer containing 80 parts by mass of UDMA and 20 parts by mass of 3G was used instead of the cationically polymerizable monomer, and the amount of the electron donating compound mixed was changed to 0.5 part by mass. The resulting photocationically curable composition was evaluated by the aforementined methods. The results are shown in Table 7.

Table 7

| | Cationically polymerizable monomer | Radically polymerizable monomer | Electron donating compound | Organic-inorganic composite filler | | | Inorganic spherical filler |
|---|---|---|---|---|---|---|---|
| | | | | Kind | Inorganic spherical filler | Organic resin matrix | |
| Example 25 | KR-470 (75) | | Th3 (0.1) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Example 26 | KR-470 (75) | | Th3 (0.3) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Example 27 | KR-470 (75) | | Th3 (0.5) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Example 28 | KR-470 (75) | | Th3 (0.5) | CF2 | F2 | D-2.6E/HD 8/92 | |
| | OXT-121(25) | | | | | | |
| | nP = 1.509 | | | (200) | $nF_b$ = 1.522 | nM = 1.509 | |
| Example 29 | KR-470 (75) | | Th3 (0.5) | CF3 | F1 | HD | |
| | OXT-121(25) | | | | | | |
| | nP = 1.509 | | | (200) | $nF_b$ = 1.522 | nM = 1.504 | |
| Example 30 | KR-470 (75) | | Th3 (0.5) | CF4 | F1 | HD/OXE-30 80/20 | |
| | OXT-121(25) | | | | | | |
| | nP = 1.509 | | | (200) | $nF_b$ = 1.522 | nM = 1.502 | |
| Example 31 | KR-470 (75) | | Th2 (0.5) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Example 32 | KR-470 (75) | | Th3 (0.5) | CF1 | F1 | D-2.6E/HD 8/92 | F1 (100) |
| | OXT-121(25) | | | | | | |
| | nP = 1.509 | | | (100) | $nF_b$ = 1.522 | nM = 1.509 | nFa = 1.522 |

(continued)

| | Cationically polymerizable monomer | Radically polymerizable monomer | Electron donating compound | Organic-inorganic composite filler | | | Inorganic spherical filler |
|---|---|---|---|---|---|---|---|
| | | | | Kind | Inorganic spherical filler | Organic resin matrix | |
| Example 33 | KR-470 (75) | | Th3 (0.5) | CF1 | F1 | D-2.6E/HD 8/92 | F1 (200) |
| | OXT-121(25) | | | | | | |
| | nP = 1.509 | | | (200) | $nF_b$ = 1.522 | nM = 1.509 | nFa = 1.522 |
| Comparative Example 17 | KR-470 (75) | | DMBE (0.5) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Comparative Example 18 | KR-470 (75) | | DMAn (0.5) | CF1 | F1 | D-2.6E/HD 8/92 | |
| | OXT-121(25) | | | | | | |
| | nP = 1.509 | | | (200) | $nF_b$ = 1.522 | nM = 1.509 | |
| Comparative Example 19 | | UDMA(80) | Th3 (0.5) | CF1 | F1 | D-2.6E/HD 8/92 | |
| | | 38(20) | | | | | |
| | | nP = 1.509 | | (200) | $nF_b$ = 1.522 | nM = 1.509 | |
| | Surface hardness (Hv) | Color tone change (ΔE*) white background | Gelation time (day) | Polymerization shrinkage rate (%) | Visual evaluation of colored light | Visual evaluation of fluorescent light | Spectral reflectance ratio |
| Example 25 | 20.3 | 2.8 | > 30 | 1.2 | yellow | - | 0.95 |
| Example 26 | 21.5 | 2.8 | > 30 | 1.2 | yellow | - | 0.99 |
| Example 27 | 22.2 | 2.9 | > 30 | 1.2 | yellow | - | 1.06 |
| Example 28 | 22.5 | 3.0 | > 30 | 1.2 | red | - | 0.90 |
| Example 29 | 18.7 | 2.8 | > 30 | 1.2 | yellow | - | 0.91 |
| Example 30 | 20.1 | 2.7 | > 30 | 1.2 | yellow | - | 1.02 |
| Example 31 | 20.2 | 3.3 | > 30 | 1.2 | yellow | - | 0.94 |
| Example 32 | 23.5 | 3.1 | > 30 | 1.3 | yellow | - | 1.08 |
| Example 33 | 38.6 | 2.5 | > 30 | 0.7 | yellow | - | 1.05 |

(continued)

| | Surface hardness (Hv) | Color tone change (ΔE*) white background | Gelation time (day) | Polymerization shrinkage rate (%) | Visual evaluation of colored light | Visual evaluation of fluorescent light | Spectral reflectance ratio |
|---|---|---|---|---|---|---|---|
| Comparative Example 17 | 6.8 | 7.6 | > 30 | 1.1 | yellow | - | 1.02 |
| Comparative Example 18 | 23.5 | 4.5 | > 30 | 1.2 | yellow | blue | 0.34 |
| Comparative Example 19 | 17.8 | 3.0 | > 30 | 2.5 | yellow | - | 0.98 |

The values in parentheses show the amounts part by mass of the components added.
nP shows the refractive index (25°C) of the polymer of the polymerizable monomer, nF shows the refractive index (25°C) of the inorganic spherical filler, and nM shows the refractive index (25°C) of the organic resin matrix.
Additional components: DPIB (2.0), CQ (0.3), HQME (0.1), BHT (0.1)

It was understood that the photocationically curable compositions of Examples 25 to 33 had strong yellowish to reddish colored light, and were capable of performing restoration with high color tone compatibility with the natural teeth. Furthermore, the photocationically curable compositions of the present invention had good properties, i.e., small polymerization shrinkage and a high surface hardness of the cured material.

On the other hand, the photocationically curable composition of Comparative Example 17 using the aromatic amine as the electron donating compound, but not using the thiophene compound, had a decreased surface hardness of the cured material.

It was understood that the photocationically curable composition of Comparative Example 18 using the anthracene compound as the electron donating compound, but not using the thiophene compound, was confirmed to show blue fluorescent light under ultraviolet light, and also had a low spectral reflectance ratio, resulting in difficulty in performing restoration with high color tone compatibility.

The photocationically curable composition of Comparative Example 19 using the radically polymerizable monomer instead of the cationically polymerizable monomer resulted in a larger polymerization shrinkage rate than Examples.

## Claims

1. A photocationically curable composition comprising

   a cationically polymerizable monomer (a),
   a photo acid generator (b),
   a photosensitizer (c), and
   a thiophene compound (d).

2. The photocationically curable composition according to claim 1, wherein a content of an electron donating compound consisting of an anthracene compound and/or an aromatic amine compound is 0.2 part by mass or less per 1 part by mass of the thiophene compound (d).

3. The photocationically curable composition according to claim 1 or 2, wherein the thiophene compound (d) is a compound having one or more $\pi$-conjugated substituents on a thiophene skeleton.

4. The photocationically curable composition according to any one of claims 1 to 3, wherein the n-conjugated substituent is one or more benzene rings or thiophene rings.

5. The photocationically curable composition according to any one of claims 1 to 4, wherein the thiophene compound (d) is a compound represented by the following formula (1) or (2):

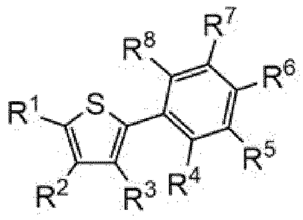

(1)

or

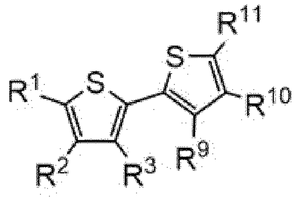

(2)

   wherein $R^1$ to $R^{11}$ each independently represent a hydrogen atom, a halogen atom, an alkyl group, an alkenyl group, an alkynyl group, an alkoxy group, an alkylthio group, an aryl group, or a thienyl group, in which $R^3$ and $R^4$, and $R^3$ and $R^9$ each may be bonded to each other to form a ring.

6. The photocationically curable composition according to any one of claims 1 to 5, wherein the cationically polymerizable monomer (a) comprises at least one kind of a compound selected from an epoxy compound and an oxetane compound, the photo acid generator (b) is an iodonium salt, and the photosensitizer (c) is a camphorquinone.

7. The photocationically curable composition according to any one of claims 1 to 6, wherein the photocationically curable composition further comprises a phenol-based antioxidant.

**8.** The photocationically curable composition according to any one of claims 1 to 7, wherein the photocationically curable composition further comprises a filling material.

**9.** The photocationically curable composition according to claim 8, wherein

the filling material contains an inorganic spherical filler ($f_1$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and
the inorganic spherical filler ($f_1$) has a refractive index $nF_a$ at 25°C that is larger than a refractive index nP at 25°C of the polymer of the cationically polymerizable monomer (a).

**10.** The photocationically curable composition according to claim 9, wherein a difference between the refractive index $nF_a$ at 25°C of the inorganic spherical filler ($f_1$) and the refractive index nP at 25°C of the polymer of the cationically polymerizable monomer (a) is 0.001 or more.

**11.** The photocationically curable composition according to any one of claims 8 to 10, wherein

the filling material contains an organic-inorganic composite filler (cf),
the organic-inorganic composite filler (cf) contains
an organic resin matrix (m) and
an inorganic spherical filler ($f_2$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and
the inorganic spherical filler ($f_2$) has a refractive index $nF_b$ at 25°C that is larger than a refractive index nM at 25°C of the organic resin matrix (m).

**12.** The photocationically curable composition according to claim 11, wherein a difference between the refractive index $nF_b$ at 25°C of the inorganic spherical filler ($f_2$) and the refractive index nM at 25°C of the organic resin matrix (m) is 0.001 or more.

**13.** The photocationically curable composition according to claim 11 or 12, wherein the refractive index $nF_b$ at 25°C of the inorganic spherical filler ($f_2$) is larger than the refractive index nP at 25°C of the polymer of the cationically polymerizable monomer (a).

**14.** The photocationically curable composition according to any one of claims 11 to 13, wherein a difference between the refractive index $nF_b$ at 25°C of the inorganic spherical filler ($f_2$) and the refractive index nP at 25°C of the polymer of the cationically polymerizable monomer (a) is 0.001 or more.

**15.** The photocationically curable composition according to any one of claims 1 to 14, wherein the photocationically curable composition comprises a radically polymerizable monomer in an amount of 10 to 70 parts by mass per 100 parts by mass of the cationically polymerizable monomer.

**16.** A dental curable composition comprising the photocationically curable composition according to any one of claims 1 to 15.

**Amended claims under Art. 19.1 PCT**

**1.** A dental curable composition comprising a photocationically curable composition including:

a polymerizable monomer component comprising a cationically polymerizable monomer (a); and
a photocationic polymerization initiator comprising a photo acid generator (b) and a photosensitizer (c), wherein the photocationically curable composition further including an electron donating compound comprising a thiophene compound (d) represented by any of the following formulae (1) to (4):

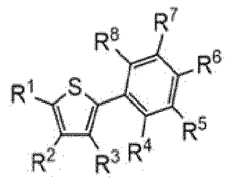

(1)

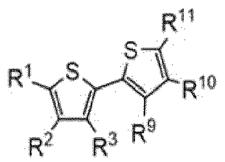

(2)

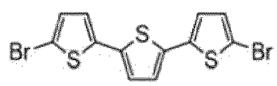

(3)

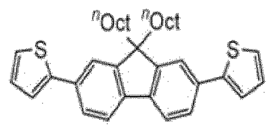

(4)

wherein in the formulae (1) and (2), $R^1$ to $R^{11}$ each independently represent a hydrogen atom, a halogen atom, an alkyl group, an alkenyl group, an alkynyl group, an alkoxy group, an alkylthio group, an aryl group, or a thienyl group, in which $R^3$ and $R^4$, and $R^3$ and $R^9$ each may be bonded to each other to form a ring, and in the formula (4), $^n$Oct represents a n-octyl group.

**2.** The dental curable composition according to claim 1, wherein a content of an electron donating compound consisting of an anthracene compound and/or an aromatic amine compound which can be included in the photocationically curable composition, is 0.2 part by mass or less per 1 part by mass of the thiophene compound (d).

**3.**

**4.**

**5.**

**6.** The dental curable composition according to claim 1 or 2, wherein the cationically polymerizable monomer (a) comprises at least one kind of a compound selected from an epoxy compound and an oxetane compound, the photo acid generator (b) is an iodonium salt, and the photosensitizer (c) is a camphorquinone.

**7.** The dental curable composition according to any one of claims 1, 2, and 6, wherein the photocationically curable composition further includes a phenol-based antioxidant.

**8.** The dental curable composition according to any one of claims 1, 2, 6, and 7, wherein the photocationically curable composition further includes a filler.

**9.** The dental curable composition according to claim 8, wherein

the filler contains an inorganic spherical filler ($f_1$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and
the inorganic spherical filler ($f_1$) has a refractive index $nF_a$ at 25°C that is larger than a refractive index nP at 25°C of the polymer of the cationically polymerizable monomer (a).

**10.** The dental curable composition according to claim 9, wherein a difference between the refractive index $nF_a$ at 25°C of the inorganic spherical filler ($f_1$) and the refractive index nP at 25°C of the polymer of the cationically polymerizable monomer (a) is 0.001 or more.

**11.** The dental curable composition according to any one of claims 8 to 10, wherein

the filler contains an organic-inorganic composite filler (cf),
the organic-inorganic composite filler (cf) contains
an organic resin matrix (m) and
an inorganic spherical filler ($f_2$) having an average primary particle diameter in a range of 230 nm or more and 1,000 nm or less and a proportion of particles existing within a range above and below 5% of the average primary particle diameter in a number-based particle size distribution of 90% or more with respect to the total number of particles, and
the inorganic spherical filler ($f_2$) has a refractive index $nF_b$ at 25°C that is larger than a refractive index nM at 25°C of the organic resin matrix (m).

**12.** The dental curable composition according to claim 11, wherein a difference between the refractive index $nF_b$ at 25°C of the inorganic spherical filler ($f_2$) and the refractive index nM at 25°C of the organic resin matrix (m) is 0.001 or more.

**13.** The dental curable composition according to claim 11 or 12, wherein the refractive index $nF_b$ at 25°C of the inorganic spherical filler ($f_2$) is larger than the refractive index nP at 25°C of the polymer of the cationically polymerizable monomer (a).

**14.** The dental curable composition according to any one of claims 11 to 13, wherein a difference between the refractive index $nF_b$ at 25°C of the inorganic spherical filler ($f_2$) and the refractive index nP at 25°C of the polymer of the cationically polymerizable monomer (a) is 0.001 or more.

**15.** The dental curable composition according to any one of claims 1 to 2 and 6 to 14, wherein the photocationically curable composition includes a radically polymerizable monomer in an amount of 10 to 70 parts by mass per 100 parts by mass of the cationically polymerizable monomer.

**16.**

**17.** The dental curable composition according to any one of claims 1 to 2 and 6 to 15, wherein contents of the photo acid generator (b), the photosensitizer (c), and the thiophene compound (d) are 0.01 to 20 parts by mass for the photo acid generator (b), 0.001 to 10 parts by mass for the photosensitizer (c), and 0.001 to 10 parts by mass for the thiophene compound (d), per 100 parts by mass of the cationically polymerizable monomer (a).

## INTERNATIONAL SEARCH REPORT

International application No. **PCT/JP2022/008296**

**A. CLASSIFICATION OF SUBJECT MATTER**

***C08G 59/68***(2006.01)i; ***C08G 65/10***(2006.01)i; ***C08G 65/18***(2006.01)i; ***A61K 6/60***(2020.01)i; ***A61K 6/62***(2020.01)i
FI: C08G65/10; C08G65/18; C08G59/68; A61K6/60; A61K6/62

According to International Patent Classification (IPC) or to both national classification and IPC

**B. FIELDS SEARCHED**

Minimum documentation searched (classification system followed by classification symbols)

C08G59/68; C08G65/10; C08G65/18; A61K6/60; A61K6/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2008-233516 A (KYOTO UNIV) 02 October 2008 (2008-10-02)<br>claims 1-6, 8, 9, paragraph [0040], examples 2, 7 | 1-5, 7, 15<br>6 |
| X<br>Y | JP 2011-501744 A (BASF SE) 13 January 2011 (2011-01-13)<br>claims 1, 9, 11, 14, 15, paragraphs [0119]-[0144], [0149]-[0152], [0214], [0232], [0241], [0246]-[0247], example 6, application example A1 | 1-8, 15, 16<br>6, 9-14 |
| Y | JP 2006-249040 A (TOKUYAMA CORP) 21 September 2006 (2006-09-21)<br>paragraphs [0035], [0037], [0074] | 6 |
| Y | JP 2006-8635 A (TOKUYAMA CORP) 12 January 2006 (2006-01-12)<br>paragraphs [0025]-[0026], [0032], [0034], [0082] | 6 |
| Y | WO 2018/194032 A1 (TOKUYAMA DENTAL CORP) 25 October 2018 (2018-10-25)<br>claims 1, 3, 5, paragraphs [0020], [0051], [0064]-[0072], examples | 9-14 |
| A | JP 2011-153212 A (SUMITOMO SEIKA CHEM CO LTD) 11 August 2011 (2011-08-11) | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 April 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/008296

C. DOCUMENTS CONSIDERED TO BE RELEVANT

| Category* | Citation of document, with indication, where appropriate, of the relevant passages | Relevant to claim No. |
|---|---|---|
| A | WO 2011/129206 A1 (SUMITOMO SEIKA CHEM CO LTD) 20 October 2011 (2011-10-20) | 1-16 |
| A | JP 2008-239519 A (SUMITOMO SEIKA CHEM CO LTD) 09 October 2008 (2008-10-09) | 1-16 |

Form PCT/ISA/210 (second sheet) (January 2015)

**INTERNATIONAL SEARCH REPORT**
**Information on patent family members**

International application No.
**PCT/JP2022/008296**

| Patent document cited in search report | Publication date (day/month/year) | Patent family member(s) | Publication date (day/month/year) |
|---|---|---|---|
| JP 2008-233516 A | 02 October 2008 | (Family: none) | |
| JP 2011-501744 A | 13 January 2011 | WO 2009/047105 A1<br>claims 1, 9, 11, 14, 15, p. 26, line 21 to p. 31, line 34, p. 32, line 23 to p. 33, line 25, p. 46, line 29 to p. 49, line 2, p. 54, lines 28-34, p. 56, lines 12-27, p. 57, lines 6-10, example 6, application example A1<br>US 2010/0297540 A1<br>KR 10-2010-0068299 A<br>CN 101952269 A<br>TW 200925145 A | |
| JP 2006-249040 A | 21 September 2006 | (Family: none) | |
| JP 2006-8635 A | 12 January 2006 | US 2005/0250868 A1<br>paragraphs [0083]-[0084], [0169], [0205]-[0206], [0566]-[0567]<br>EP 1591098 A1 | |
| WO 2018/194032 A1 | 25 October 2018 | US 2020/0121564 A1<br>claims 1, 3, 5, paragraphs [0022], [0172], [0185]-[0195], examples<br>EP 3613779 A1<br>AU 2018254066 A<br>CA 3059479 A<br>CN 110431156 A<br>KR 10-2019-0132998 A<br>BR 112019020687 A<br>RU 2019134976 A | |
| JP 2011-153212 A | 11 August 2011 | (Family: none) | |
| WO 2011/129206 A1 | 20 October 2011 | (Family: none) | |
| JP 2008-239519 A | 09 October 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H10508067 A
- JP H11130945 A
- JP 2001520758 A
- JP 2005523348 A
- WO 2011115007 A
- WO 2013039169 A
- JP S58110414 A
- JP S58156524 A


### Non-patent literature cited in the description


- **JOE D. OXMAN ; DWIGHT W. JACOBS ; MATTHEW C. TROM ; VISHAL SIPANI ; BETH FICEK ; ALEC B. SCRANTON.** Evaluation of Initiator Systems for Controlled and Sequentially Curable Free-Radical/Cationic Hybrid Photopolymerizations. *Journal of Polymer Science: Part A: Polymer Chemistry,* 2005, vol. 43, 1747-1756
- **TOMOTAKA TSUCHIMURA.** *Journal of Synthetic Organic Chemistry, Japan,* 2020, vol. 78, 41-54